# FASCICULE DE BREVET EUROPEEN

(11) **EP 0 621 255 B1**
(45) Date de publication et mention de la délivrance du brevet: **20.08.1997**
(21) Numéro de dépôt: 94400845.7
(22) Date de dépôt: 19.04.1994
(51) Int. Cl.: C07C 59/68, C07C 321/28, C07D 311/72, A61K 31/35, A61K 31/19, A61K 31/215

(54) **Nouveaux acides et esters phénoxy isobutyriques substitués**
Substituierte Phenoxy-isobuttersäuren und ihre Ester
Substituted phenoxy-isobutyric acids and esters

(30) Priorité: 20.04.1993 FR 9304606
(43) Date de publication de la demande: 26.10.1994
(73) Titulaire: ADIR ET COMPAGNIE, 92415 Courbevoie Cédex (FR)
(72) Inventeur: Regnier, Gilbert, F-92290 Chatenay Malabry (FR); Guillonneau, Claude, F-92140 Clamart (FR); Vilaine, Jean-Paul, F-92290 Chatenay Malabry (FR); Lenaers, Albert, F-78510 Triel Sur Seine (FR); Breugnot, Christine, F-75015 Paris (FR)
(74) Mandataire: Reverbori, Marcelle

(56) Documents cités:
- EP-A- 0 407 200
- DE-A- 1 493 973
- FR-A- 2 348 182
- GB-A- 1 288 841
- GB-A- 1 394 260
- US-A- 4 039 683

## Description

La présente invention a pour objet les nouveaux acides et esters phénoxy isobutyriques substitués, leur procédé de préparation et les compositions pharmaceutiques les renfermant.

Elle concerne plus particulièrement les acides et esters phénoxy isobutyriques substitués de formule I : 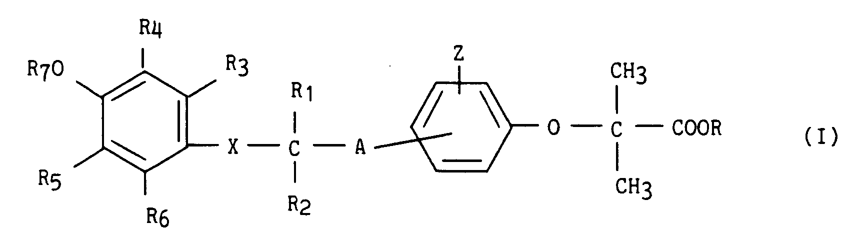 dans laquelle :
- X représente un atome d'oxygène, un atome de soufre ou une liaison simple ;
- A représente une liaison simple ou un radical hydrocarboné contenant de 1 à 9 atomes de carbone en chaîne droite ou ramifiée renfermant éventuellement une double liaison, un radical cyclopropyle, un atome d'oxygène ou un radical carbonyle, ou éventuellement substitué par un atome d'halogène (tel que par exemple chlore ou brome) ou un radical hydroxy ;
- R représente un atome d'hydrogène ou un radical alkyle de 1 à 6 atomes de carbone en chaîne droite ou ramifiée éventuellement substitué par un ou deux radicaux hydroxy ;
- R₁ et R₃ :
   . représentent chacun simultanément un atome d'hydrogène, ou
   . forment ensemble un pont (CH₂)ₙ dans lequel n prend les valeurs 1 ou 2, ou
   . R₁ représente :
      . un radical méthyle, ou
      . une liaison simple formant une double liaison avec le groupe A lorsque celui-ci est un radical hydrocarboné, et
      . dans chacun de ces cas, simultanément R₃ représente un atome d'hydrogène ;
- R₂ et R₆, identiques ou différents, représentent chacun un atome d'hydrogène ou un radical méthyle ;
- R₄ et R₅, identiques ou différents, représentent chacun un radical alkyle de 1 à 6 atomes de carbone en chaîne droite ou ramifiée ;
- R₇ représente un atome d'hydrogène ou un groupement protecteur labile tel que par exemple un radical CH₃CO-, C₂H₅O-CH₂- ou benzyle ; et
- Z représente un atome d'hydrogène ou d'halogène ou un radical alkyle ou alkoxy contenant chacun de 1 à 5 atomes de carbone en chaîne droite ou ramifiée.

Certains des composés de formule I comportent un ou plusieurs atomes chiraux et peuvent ainsi exister sous forme d'énantiomères ou de diastéréoisomères qui font également partie de la présente invention.

De même, les composés de formule I dans laquelle R représente un atome d'hydrogène peuvent être transformés en sels d'addition avec des bases pharmaceutiquement acceptables, sels qui sont, à ce titre, inclus dans la présente invention.

L'état antérieur de la technique le plus proche de la présente invention est illustré par le brevet US 4,752,616 qui a pour objet, entre autres, des acides et esters thioalkylphényl alcanoïques de formule : 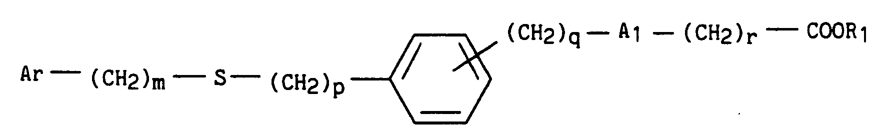 dans laquelle :
- Ar représente, entre autres, un radical phényle éventuellement substitué ;
- A₁ est un groupe de formule : 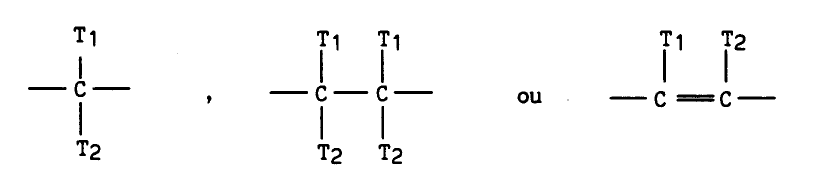 (T₁ et T₂ étant hydrogène ou alkyle inférieur) ;
- m est zéro, 1, 2 ou 3 ;
- p est un nombre entier de 1 à 5 ;
- q est zéro, 1, 2 ou 3 ;
- r est zéro, 1 ou 2, et
- R₁ est hydrogène, alkyle inférieur ou un métal alcalin.

Ces dits composés sont des agents anti-thrombotiques, anti-asthmatiques et vasodilatateurs.

Les composés de la présente invention diffèrent des composés antérieurement connus ci-dessus définis, à la fois par leur structure chimique et par leur activité pharmacologique et thérapeutique découlant de leur effet anti-oxydant mis en évidence vis à vis des LDL humaines (lipoprotéines de faible densité), et de leur effet hypolipémiant potentiel.

La présente invention a également pour objet le procédé de préparation des composés de formule I caractérisé en ce que l'on fait réagir :
a)
   . soit un composé de formule IIa : 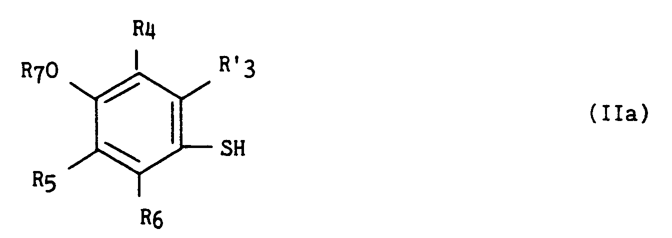 dans laquelle :
      - R₄, R₅, R₆ et R₇ ont les significations précédemment définies, et
      - R'₃ représente un atome d'hydrogène
   . avec un composé de formule IIIa : 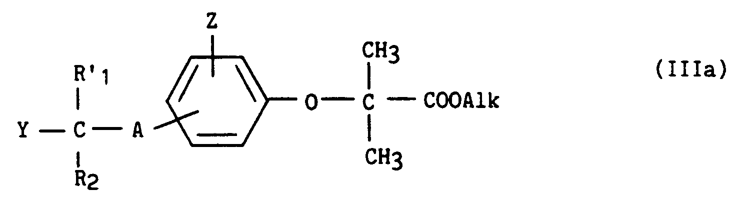 dans laquelle :
      - R₂, A et Z ont les significations précédemment définies ; - R'₁ représente un atome d'hydrogène ou un radical méthyle ;
      - Alk représente un radical alkyle de 1 à 6 atomes de carbone en chaine droite ou ramifiée,
         et
      - Y représente un atome de chlore ou de brome ;
   . pour obtenir un composé de formule Ia₁ : 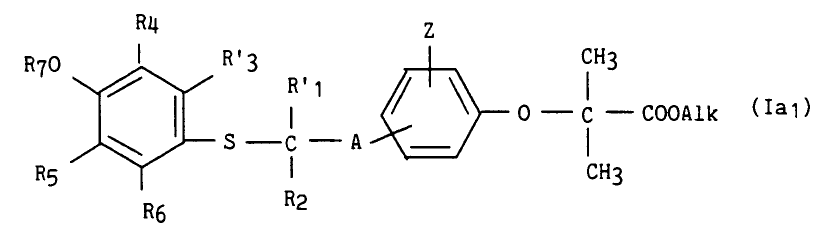 dans laquelle R'₁, R₂, R'₃, R₄, R₅, R₆, R₇, A, Z et Alk ont les significations précédemment définies,
      lequel composé Ia₁ est, selon la nature de R₇, transformé, par des méthodes séquentielles de saponification, hydrolyse ou hydrogénolyse, en un composé de formule Ia₂ : 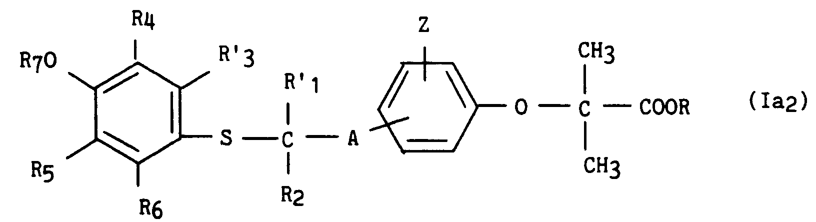 dans laquelle R'₁, R₂, R'₃, R₄, R₄, R₆, R₇, A, Z et R ont les significations précédemment définies,
b)
   . soit un composé de formule IIb : 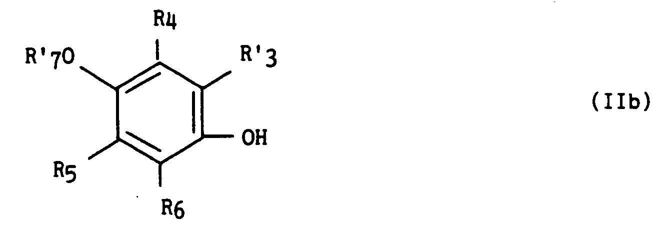 dans laquelle :
      - R'₃, R₄, R₅ et R₆ ont les significations précédemment définies et
      - R'₇ représente un groupement protecteur labile tel que : CH₃CO-, C₂H₅-O-CH₂- ou benzyle ;
   . avec un composé de formule IIIb : 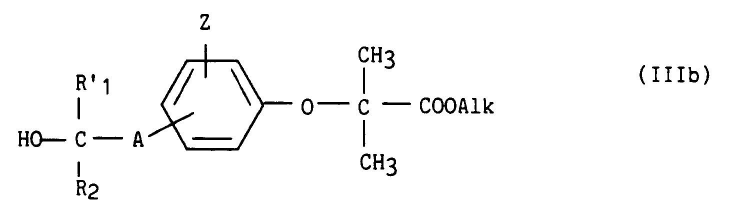 dans laquelle R'₁, R₂, A, Z et Alk ont les significations précédemment définies,
   . pour obtenir un composé de formule Ib₁ : 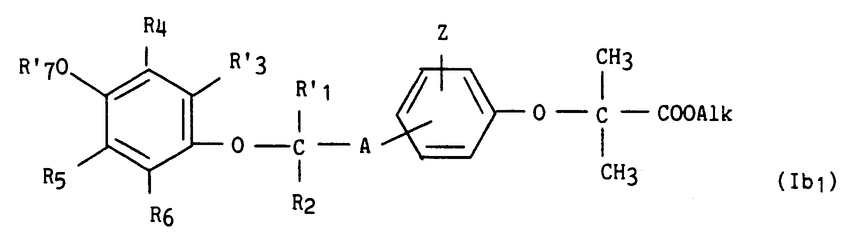 dans laquelle R'₁, R₂, R'₃, R₄, R₅, R₆, R'₇, A, Z et Alk ont les significations précédemment définies,
   . lequel composé de formule Ib₁ est, selon la nature de R'₇, transformé par des méthodes séquentielles de saponification, hydrolyse ou hydrogénolyse en un composé de formule Ib₂ : 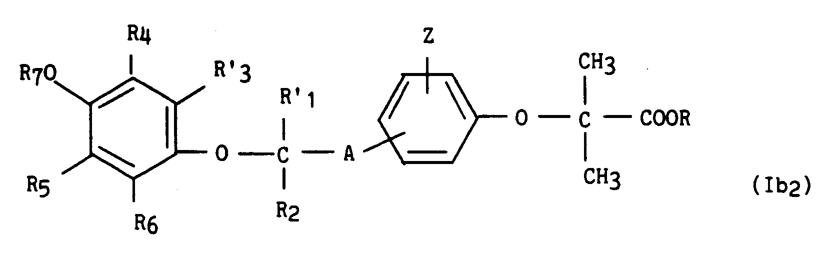 dans laquelle R'₁, R₂, R'₃, R₄, R₅, R₆, R₇, A, Z et R ont les significations précédemment définies ;
c)
   . soit un composé de formule IIc : 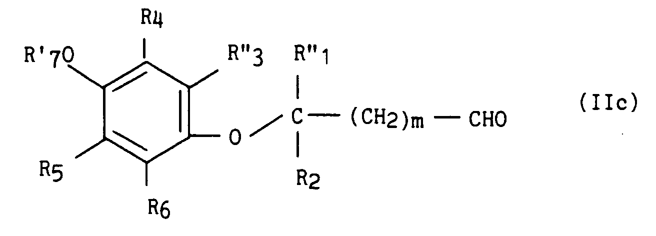 dans laquelle :
      - R₂, R₄, R₅, R₆ et R'₇ ont les significations précédemment définies,
      - R''₁ et R''₃ représentent ensemble un pont (CH₂)ₙ dans lequel n prend la signification précédemment définie, et
      - m représente zéro ou un nombre entier de 1 à 5 ;
   . avec un composé de formule IIIc : 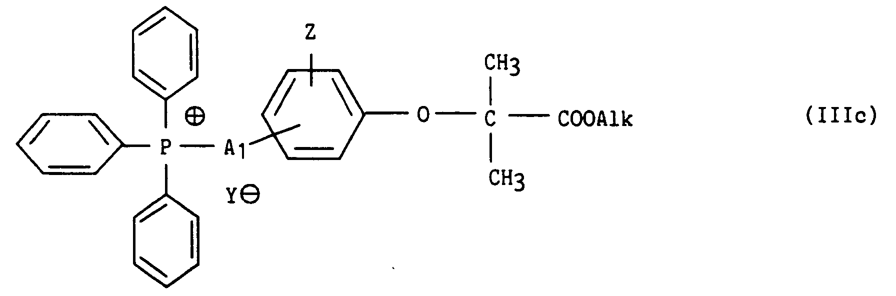 dans laquelle :
      - Alk et Z et Y ont les significations précédemment définies,
      - A₁ représente une liaison simple où un radical hydrocarboné contenantde 1 à 3 atomes de carbone en chaîne droite ou ramifiée ;
   . pour obtenir un composé de formule Ic₁ : 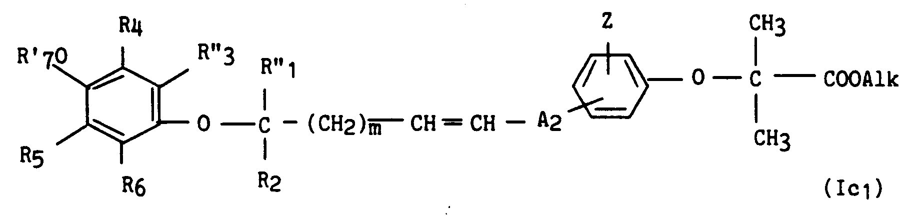 dans laquelle :
      - R''₁, R₂, R''₃, R₄, R₅, R₆, R'₇, m, Z et Alk ont les significations précédemment définies, et
      - A₂ représente une liaison simple ou un radical hydrocarboné contenant 1 ou 2 atomes de carbone en chaîne droite ou ramifiée ;
         lequel composé de formule Ic₁ est réduit pour obtenir le composé ce formule Ic₂ : 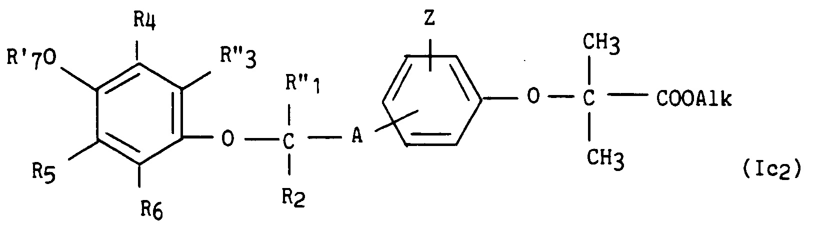 dans laquelle R''₁, R₂, R''₃, R₄, R₅, R₆, R'₇, A, Z et Alk ont les significations précédemment définies ;
         lequel composé de formule Ic₂ est, selon la nature de R'₇, transformé par des méthodes séquentielles de saponification, hydrolyse ou hydrogénolyse en un composé de formule Ic₃ : 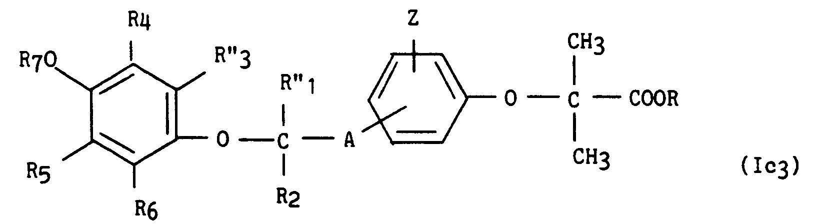 dans laquelle R''₁, R₂, R''₃, R₄, R₅, R₆, R₇, A, Z et R ont les significations précédemment définies.
d) soit un composé de formule II d) : 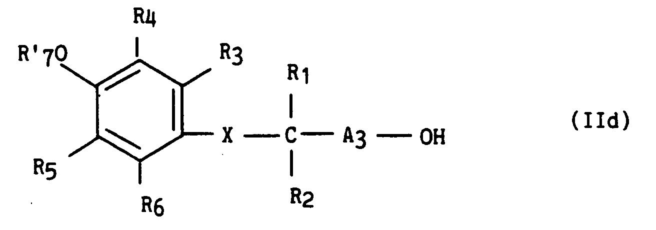 dans laquelle :
   - R₁, R₂, R₃, R₄, R₅, R₆, R'₇ et X ont les significations précédemment définies, et
   - A₃ représente une liaison simple ou un radical hydrocarboné contenant de 1 à 9 atomes de carbone en chaîne droite ou ramifiée,
      avec un composé de formule IIId : 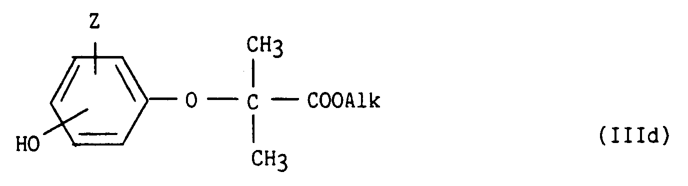 dans laquelle Z et Alk ont les significations précédemment définies,
      pour obtenir un composé de formule Id₁ : 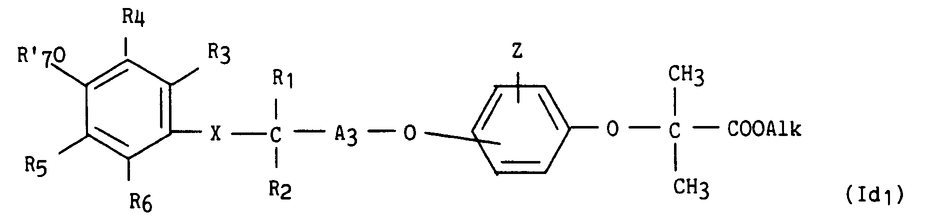 dans laquelle R₁, R₂, R₃, R₄, R₅, R₆, R'₇, X, A₃ et Alk ont les significations précédemment définies;
      lequel composé de formule Id₁ ci-dessus défini peut être saponifié, hydrogénolysé ou hydrolysé pour donner l'acide correspondant de formule Id₂ : 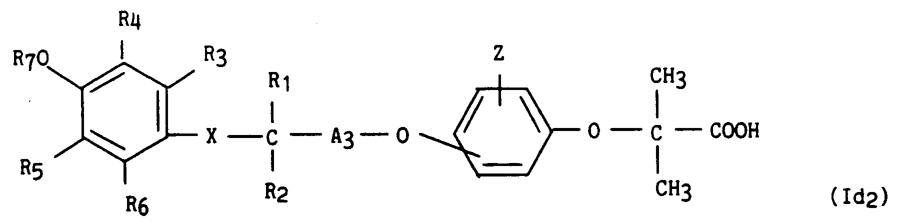 dans laquelle R₁, R₂, R₃, R₄, R₅, R₆, R₇, X, A₃ et Z ont les significations précédemment définies ;
e) soit un composé de formule IIe : 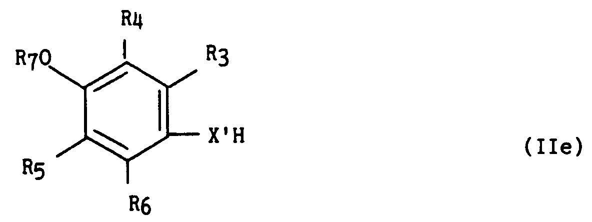 dans laquelle :
   - R₃, R₄, R₅, R₆ et R₇ ont les significations précédemment définies, et
   - X' représente un atome d'oxygène ou de soufre,
      avec un composé de formule IIIe : 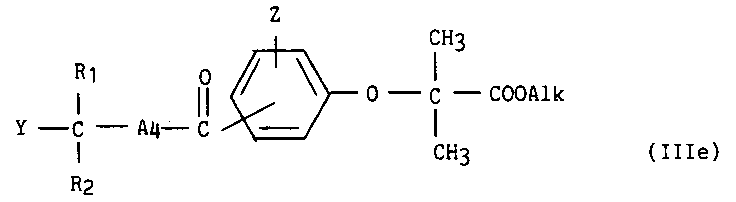 dans laquelle :
   - Y, R₁, R₂, Z et Alk ont les significations précédemment définies, et
   - A4 représente une liaison simple ou un radical hydrocarboné ayant de 1 à 8 atomes de carbone en chaîne droite ou ramifiée ;
      pour obtenir un composé de formule Ie₁ : 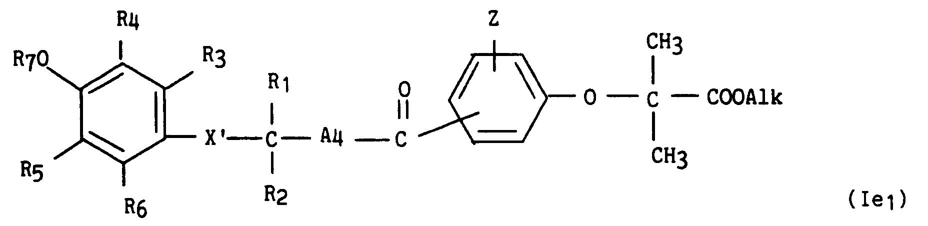 dans laquelle R₁, R₂, R₃, R₄, R₅, R₆, R₇, X', A₄, Z et Alk ont les significations précédemment définies ;
      lequel composé de formule Ie₁ ci-dessus défini peut être saponifié pour donner l'acide correspondant de formule Ie₂ : 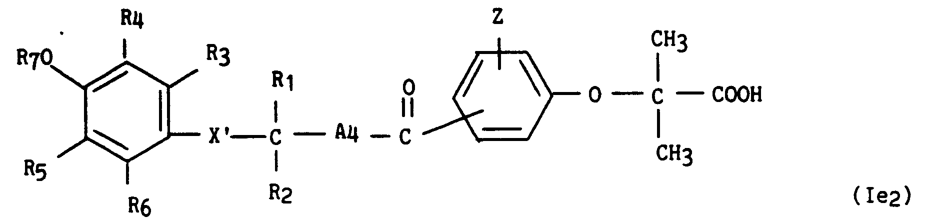 dans laquelle R₁, R₂, R₃, R₄, R₅, R₆, R₇, X', A₄ et Z ont les singifications précédemment définies ;
f) soit le composé de formule Ie₁ précédemment défini avec un réducteur chimique tel que par exemple le borohydrure de sodium ou avec de l'hydrogène en présence d'un catalyseur d'hydrogénation pour obtenir le composé de formule If₁ : 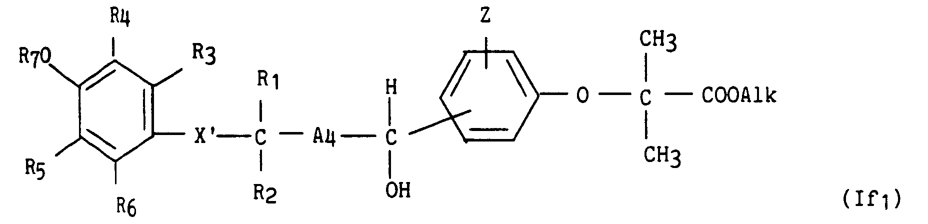 dans laquelle :
   - R₁, R₂, R₃, R₄, R₅, R₆, R₇, X', A₄, Z et Alk ont les significations précédemment définies ;
      lequel composé de formule If₁ ci-dessus défini peut être saponifié pour donner l'acide correspondant de formule If₂ : 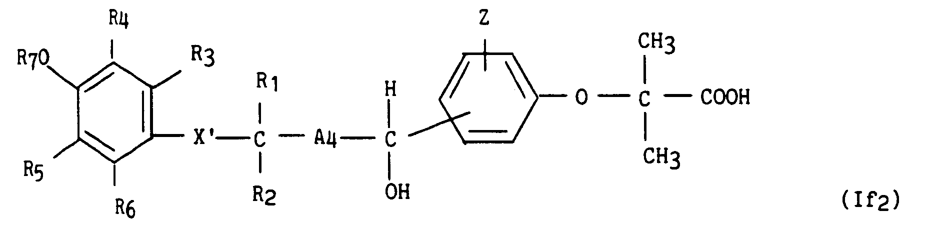 dans laquelle R₁, R₂, R₃, R₄, R₅, R₆, R₇, X', A₄ et Z ont les significations précédemment définies ;
g) soit un composé de formule If₁ ci-dessus défini,
   avec un hydracide de formule IV :

   H-Y (IV)

   dans laquelle Y a la signification précédemment définie,
   pour obtenir un composé de formule Ig₁ : 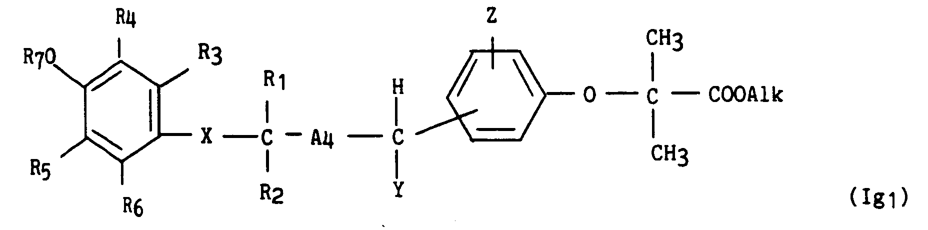 dans laquelle R₁, R₂, R₃, R₄, R₅, R₆, R₇, A₄, Y, Z et Alk ont les significations précédemment définies,
   lequel composé Ig₁ ci-dessus défini est hydrolysé pour donner l'acide correspondant de formule Ig₂ : 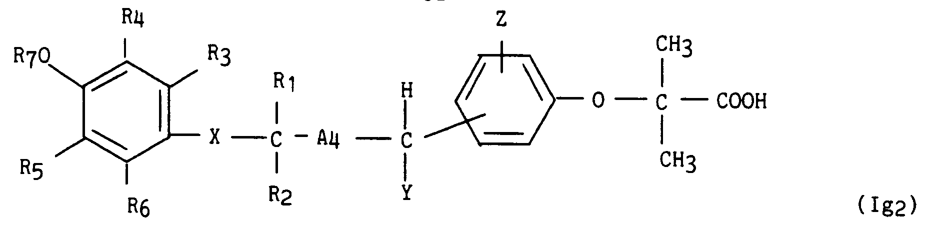 dans laquelle R₁, R₂, R₃, R₄, R₅, R₆, R₇, A₄, Y et Z ont les significations précédemment définies.

L'ensemble des composés de formule Ia₁, Ia₂, Ib₁, Ib₂, Ic₁, Ic₂, Ic₃, Id₁, Id₂, Ie₁, Ie₂, If₁, If₂, Ig₁, Ig₂ forme l'ensemble des composés de formule I.

Il est particulièrement avantageux de faire réagir les composés de formules respectives IIa et IIIa en présence d'un accepteur de l'hydracide formé au cours de la réaction, dans un solvant tel que par exemple l'acétone, l'acétonitrile ou le diméthylformamide, à une température comprise entre 50 et 120 °C.

Comme accepteur, on peut utiliser par exemple un carbonate alcalin en présence d'un iodure alcalin, la diméthylaminopyridine ou la triéthylamine.

La réaction des composés de formules respectives IIb et IIIb s'effectue selon la technique de O. Mitsunobu, Synthesis (1981),1-28, en utilisant comme réactifs l'azodicarboxylate d'éthyle et la triphénylphosphine et en opérant dans un solvant aprotique tel que par exemple le tetrahydrofurane ou l'éther à une température comprise entre 20 et 25 °C.

La réaction des composés de formules respectives IIc et IIIc s'effectue avantageusement selon la technique de Wittig. G, Ann (1953), 580, 44 et Bruce et coll., Chem. Rev.(1989), 863-927, en utilisant le butyl lithium comme réactif et en opérant en milieu tétrahydrofurane, à une température comprise entre 20 et 25 °C.

Une variante de cette technique, qui conduit à des rendements supérieurs, s'effectue selon Buddrus, Chem. Ber. (1974), 107, 2050-61. On opère dans ce cas en présence de 1,2-époxybutane en excès qui sert à la fois de réactif et de solvant, à la température de reflux (63 °C).

L'hydrogénation catalytique du composé Ic₁ s'effectue au moyen de charbon palladié sous une pression de 5.10⁵ Pa, en opérant dans l'éthanol a une température comprise entre 20 et 50 °C.

Les matières premières de formules IIa et IIb sont des produits commerciaux déjà décrits dans la littérature.

Les matières premières de formule IIIb sont obtenues, sous forme huileuse, selon le procédé qui consiste à faire réagir un composé de formule : 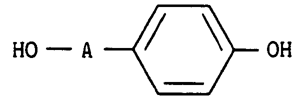 dans laquelle A a la signification précédemment définie,
avec un excès d'un composé de formule : 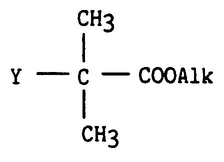 dans laquelle Y et Alk ont les significations précédemment définies ; la réaction s'effectuant dans un solvant approprié comme, par exemple, la 3-méthylpentanone, à une température voisine de 118 °C en présence d'un accepteur de l'hydracide formé au cours de la réaction, tel que par exemple le carbonate de potassium en présence d'iodure de potassium.

Les matières premières de formule IIIa sont obtenues sous forme huileuse, en faisant réagir les composés de formule IIIb avec la triphénylphosphine en présence de CCl₄ ou de brome dans l'acétonitrile, selon la méthode de J. HOOZ et coll., Can. J. Chem. 46, 86-7 (1968) ou de J. Schaefer et coll., Org. Synth. coll. Vol V, 249.

Les matières premières de formule IIc sont décrites dans la littérature et préparées selon N. Cohen et coll., J. Am. Chem. Soc. 101, 6710-15 (1979) ou selon Takeda, E.P. 345 593.
Les matières premières de formule IIIc sont préparées sous forme non cristallisée, amorphe, selon le procédé classique qui consiste à faire réagir un composé de formule IIIa telle que précédemment définie, avec la triphénylphosphine, dans l'acétonitrile à reflux pendant 20 heures.

Les matières premières de formule respective IId et IIId ont été préparées, en utilisant des réactions usuelles, à partir des phénols correspondantes convenablement protégés.

Les matières premières de formule IIIe ont été préparées à partir de cétophénols commerciaux par des méthodes classiques usuelles.

La réduction du composé de formule Ie₁ par un réducteur chimique peut être effectuée dans un solvant tel que le méthanol, l'éthanol, le diméthylformamide ou le tétrahydrofurane. L'hydrogénation catalytique du composé de formule Ie1 peut être effectuée en utilisant comme catalyseur du palladium sur charbon, de l'hydroxyde de palladium sur charbon, du platine sur charbon ou du nickel de Raney.

Les dérivés de formule I ainsi obtenus peuvent être purifiés par chromatoflash sur silice (35-70µ) en utilisant comme éluant H₃C-COOC₂H₅ ou CH₂Cl₂/CH₃OH par exemple, ou par formation de sels et cristallisation de ceux-ci.

Certains dérivés de formule I donnent des sels avec des bases physiologiquement tolérables -sels qui sont à ce titre, inclus dans la présente invention.

Les dérivés de la présente invention possèdent des propriétés pharmacologiques et thérapeutiques intéressantes.

En particulier, pour ces dérivés, a été mise en évidence in vitro et ex-vivo leur capacité à protéger les LDL humaines (lipoprotéines de faible densité, assurant le transport du cholestérol) vis à vis des modifications oxydatives induites par le cuivre et par les cellules endothéliales.

Les modifications oxydatives des LDL apparaissent actuellement constituer un mécanisme important de la formation et de l'extension des lésions vasculaires athéromateuses. Aussi les propriétés anti-oxydantes, notamment au niveau des LDL, des dérivés de la présente invention permettent leur utilisation comme médicament dans le traitement :
- des hypercholestérolémies,
- des hypertriglycéridémies,
- des dyslipémies et du diabète, pour prévenir les complications notamment vasculaires,
- de l'athérosclérose avec ses différentes localisations : vasculaires, périphériques, coronaires ou cérébrales,
- mais aussi dans les pathologies dans lesquelles une peroxydation lipidique membranaire joue un rôle initiateur et/ou aggravant telles les cardiopathies ischémiques, la reperfusion d'organes, y compris transplantés, les pathologies ischémiques traumatiques ou dégénératives du système nerveux central ou périphérique, les maladies inflammatoires aigües ou chroniques et les maladies auto-immunes.

La présente invention a également pour objet les compositions pharmaceutiques contenant comme principe actif un composé de formule I ou un de ses sels physiologiquement tolérable mélangé ou associé à un excipient pharmaceutique approprié comme, par exemple, le glucose, le lactose, l'amidon, le talc, l'éthylcellulose, le stéarate de magnésium ou le beurre de cacao.

Ces compositions pharmaceutiques se présentent généralement sous forme dosée et peuvent contenir de 5 à 250 mg de principe actif.

Elles peuvent revêtir, par exemple, la forme de comprimés, dragées, gélules, suppositoires, solutions injectables ou buvables, et être selon les cas, administrées par voie orale, rectale ou parentérale à dose de 5 à 500 mg en 1 ou 2 prises quotidiennes.

Les exemples suivants illustrent la présente invention, les points de fusion étant déterminés à la platine chauffante de Kofler (K) ou au tube capillaire (cap).

### Exemple 1

Acide 2-{4-[2-(3,5-di-tert-butyl-4-hydroxyphénylthio)éthyl]phénoxy} isobutyrique

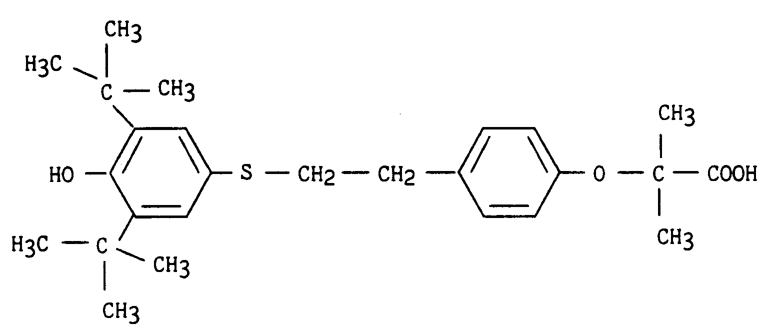

Dans un tricol muni d'une agitation, d'un réfrigérant et placé sous atmosphère d'azote, on introduit 5,55 g (0,0176 mole) de 2-[4-(2-bromoéthyl)phénoxy]isobutyrate d'éthyle, 4,25 g (0,025 mole) de 4-hydroxy-3,5-ditert-butyl phénylthiol, 2,43 g de carbonate disodique, 125 ml d'acétone et 1 g d'iodure de potassium. On porte l'ensemble au reflux que l'on maintient pendant 20 heures. On concentre à sec et reprend le résidu dans le chlorure de méthylène et l'eau.

Après décantation, on sèche la phase organique sur sulfate de sodium et concentre à sec, puis on chromatographie sur 700 cm³ de silice Amicon (0,035-0,070 mm) en éluant avec un mélange de cyclohexane et de chlorure de méthylène (70-30).

On concentre les phases intéressantes et obtient 7,8 g de l'ester attendu sous forme huileuse. Rendement 94 %.

Dans un tricol, muni d'un agitateur, d'un réfrigérant et sous atmosphère d'azote, on introduit 7,6 g (0,016 mole) de l'ester ci-dessus obtenu, 300 ml d'éthanol et 18 ml de solution normale d'hydroxyde de sodium. L'ensemble est porté à reflux pendant 12 heures, puis concentré à sec.

Le résidu est repris dans un mélange d'éther éthylique et d'eau. On extrait plusieurs fois la phase aqueuse à l'éther éthylique. Les phases éthérées jointes sont séchées sur sulfate de sodium. On observe une cristallisation. On essore et reprend les cristaux dans 100 ml d'acide chlorhydrique normal et 200 ml d'éther éthylique. On agite vivement, décante, lave la phase organique avec une solution saturée de chlorure de sodium, sèche sur sulfate de sodium et concentre à sec.

Le résidu gommeux est repris par 30 ml de cyclohexane à reflux. On refroidit et observe une cristallisation. On essore, lave au cyclohexane froid, sèche à 60 °C sous 67 Pa et obtient 5,4 g d'acide 2-{4-[2-(3,5-di-tert-butyl-4-hydroxyphénylthio)éthyl]phénoxy}isobutyrique sous forme de cristaux blancs fondant (K) à 100 °C. Rendement 76 %.

### Exemples 2-8

En opérant comme décrit dans l'exemple 1 ont été préparés les composés objet des exemples suivants :
2) le 2-{4-[2-(4-hydroxy-2,3,5-triméthylphénylthio)éthyl]phénoxy} isobutyrate d'éthyle (gomme).
3) l'acide 2-{4-[2-(4-hydroxy-2,3,5-triméthylphénylthio)éthyl]phénoxy} isobutyrique, P.F. (K) : 70 °C (acétate d'éthyle).
4) l'acide 2-[4-(3,5-di-tert-butyl-4-hydroxyphénylthiométhyl)phénoxy] isobutyrique, P.F. (K) : 134 °C (CH₂Cl₂/acétone).
5) le sel de tert-butylamine de l'acide 2-{4-[3-(3,5-ditert-butyl-4-hydroxyphénylthio)propyl]phénoxy}isobutyrique, P.F. (K) : 124 °C (éther de pétrole).
6) le sel de tert-butylamine de l'acide 2-{4-[5-(3,5-di-tert-butyl-4-hydroxyphénylthio)pentyl]phénoxy}isobutyrique, P.F. (cap) : 106-110 °C (Pentane).
7) Le 2-{4-chloro-3-[2-(4-hydroxy-2,3,5-triméthylphénylthio)éthyl]phénoxy} isobutyrate de sodium (lyophilisat).
8) L'acide 2-{4-chloro-3-[2-(3,5-ditert-butyl-4-hydroxyphénylthio)éthyl] phénoxy}isobutyrique, PF (cap) : 104-107 °C.

### Exemple 9

Acide 2-{4-[2-(4-hydroxy-2,3,5-triméthylphénoxy)éthyl]phénoxy}isobutyrique

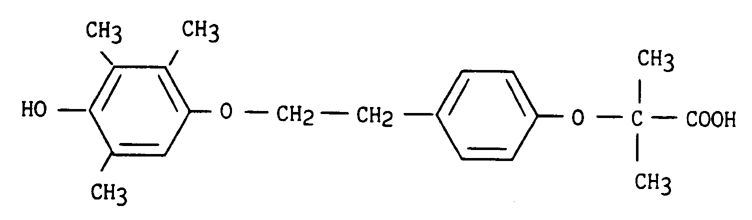

Dans un tricol muni d'une agitation, d'un réfrigérant, d'une ampoule à brome et d'un thermomètre, sous atmosphère d'azote, on introduit 11 g (0,0567 mole) de 4-acétoxy-2,3,5-triméthylphénol, fondant (K) à 108 °C, 16,3 g de triphénylphosphine et 300 ml de tétrahydrofurane. On refroidit à 5 °C et coule, en 15 minutes sous agitation en maintenant la température inférieure à 10 °C, 10,65 g d'azodicarboxylate d'éthyle en solution dans 75 ml de tétrahydrofurane. On agite pendant 30 minutes à 5 °C et coule 15,5 g de 2-[4-(2-hydroxyéthyl)phénoxy]isobutyrate d'éthyle en solution dans 100 ml de tétrahydrofurane, sans dépasser 5 °C. On agite pendant 1 heure à 5 °C puis 20 heures à température ambiante. On concentre à sec, triture le résidu dans le cyclohexane, filtre et concentre à sec le filtrat.

On chromatographie le concentrat sur 2,7 l de silice Amicon (0,035-0,070 mm) en éluant avec du chlorure de méthylène. On concentre les fractions intéressantes et obtient 10,7 g de l'ester attendu sous forme d'un produit gommeux. Rendement : 44 %.

10,5 g (0,0245 mole) du 2-{4-[2-(4-acetoxy-2,3,5-triméthylphénoxy)éthyl] phénoxy}isobutyrate d'éthyle ainsi obtenu sont introduits avec 500 ml d'éthanol et 50 ml de solution normale de soude dans un tricol muni d'une agitation, d'un réfrigérant et placé sous atmosphère d'azote. L'ensemble est porté à reflux pendant 3 heures. Puis on concentre à sec, reprend le résidu dans l'eau et extrait plusieurs fois à l'éther. La phase aqueuse est acidifiée par 55 cm³ d'acide chorhydrique normal puis extraite plusieurs fois à l'éther. Les phases éthérées jointes sont lavées à l'eau, séchées sur sulfate de soude et concentrées à sec. Le résidu est chromatographié sur 1,4 l de silice Amicon (0,035-0,070 mm) en éluant avec un mélange de chlorure de méthylène et d'acétate d'éthyle (70-30). Les fractions intéressantes sont concentrées à sec. Le résidu est dissous dans 20 ml d'éther. On dilue à l'éther de pétrole et refroidit. On observe une cristallisation. On essore les cristaux et les sèche à 45 °C sous 133 Pa. On obtient 6 g d'acide 2-{4-[2-(4-hydroxy-2,3,5-triméthylphénoxy)éthyl]phénoxy}isobutyrique, sous forme de cristaux blancs, fondant (K) à 69 °C. Rendement : 68 %.

### Exemples 10-14

En opérant comme décrit dans l'exemple 9, ont été préparés les composés objet des exemples suivants :
10) l'acide 2-{4-[2-(3,5-di-tert-butyl-4-hydroxyphénoxy)éthyl]phénoxy} isobutyrique, P.F (cap) : 119-120°C. (CH₂Cl₂/acétone).
11) le sel de tert-butylamine de l'acide 2-{4-[5-(3,5-di-tert-butyl-4-hydroxyphénoxy)pentyl]phénoxy}isobutyrique, P.F (cap) : 132-133 °C (pentane).
12) le 2-{4-[2-(4-acétoxy-2,3,5-triméthylphénoxy)éthyl]phénoxy} isobutyrate d'éthyle (gomme).
13) le 2-{4-chloro-3-[2-(4-hydroxy-2,3,5-triméthylphénoxy)éthyl]phénoxy} isobutyrate de sodium, (lyophilisat).
14) le 2-{4-chloro-3-[2-(3,5-ditert-butyl-4-hydroxyphénoxy)éthyl]phénoxy} isobutyrate de tert-butylamine, PF (cap) : 160-163 °C (éther/éther de pétrole).

### Exemple 15

(Z),(R,S)-2-{4-[3-(6-hydroxy-2,5,7,8-tétraméthylchroman-2-yl)prop-2-én-1-yl] phénoxy}isobutyrate de tert-butylamine.

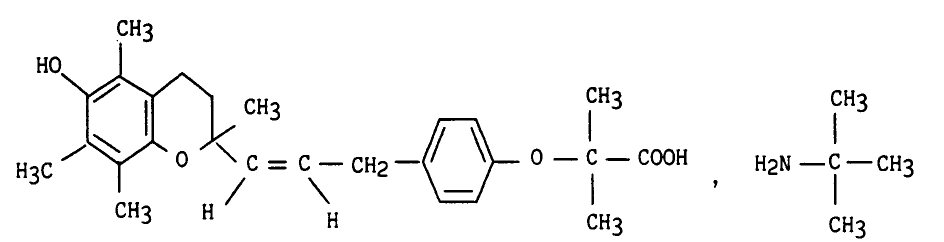

Dans un ballon muni d'une agitation et d'un réfrigérant on introduit 30 g (0,052 mole) de bromure de 2-{4-[2-(éthoxycarbonyl)propan-2-yl oxy] phényl}éthyl triphényl phosphonium, 15,2 g (0,052 mole) de 6-éthoxyméthoxy-2-formyl-2,5,7,8-tétraméthyl chromane, fondant (K) à 66 °C, et 1,8 l de 1,2-époxybutane. On porte au reflux pendant 48 heures. On concentre à sec, reprend le résidu dans le toluène et concentre à nouveau.

On chromatographie le résidu sur 2 l de silice Amicon (0,035-0,070 mm) en éluant avec un mélange de chloroforme (stabilisé à l'amylène) et d'acétate d'éthyle (96-4). Les fractions intéressantes sont concentrées à sec. On obtient 21,2 g de (Z),(R,S)-2-{4-[3-(6-éthoxyméthoxy-2,5,7,8-tétra méthylchroman-2-yl)prop-2-én-1-yl]phénoxy}isobutyrate d'éthyle, sous forme de gomme. Rendement 80 %.

Le 6-éthoxyméthoxy-2-formyl-2,5,7,8-tétraméthyl chromane de départ a été préparé par réduction, à l'hydrure de diisobutyl aluminium de l'ester méthylique correspondant (huile n_{D}^{20°C} : 1,5207) lui-même préparé à partir de l'ester méthylique du Trolox et du chlorométhyl éthyléther dans le diméthylformamide en présence de HNa.

Dans un ballon muni d'une agitation et d'un réfrigérant, on introduit 5,1 g (0,01 mole) de (Z),(R,S)-2-{4-[3-(6-éthoxyméthoxy-2,5,7,8-tétraméthyl chroman-2-yl)prop-2-én-1-yl]phénoxy}isobutyrate d'éthyle ci-dessus préparé, 100 ml d'éthanol et 20 ml d'acide chlorhydrique normal. On porte au reflux pendant 3 heures et abandonne une nuit à température ambiante. On concentre à sec, reprend le résidu dans l'éther éthylique, lave avec une solution saturée de chlorure de sodium, sèche sur sulfate de sodium et concentre à sec. Le résidu est repris dans 200 ml d'éthanol auquel on ajoute 20 ml de soude N. On abandonne 20 heures à température ambiante et concentre à sec. Le résidu est repris par 21 ml d'HCl/N. On extrait à l'éther éthylique plusieurs fois. Les phases éthérées sont lavées avec une solution saturée de chlorure de sodium puis séchées sur sulfate de sodium. On concentre à sec. Le résidu est chromatographié sur 500 ml de silice Amicon (0,035-0,070 mm) en éluant avec un mélange de CH₂Cl₂ et d'acétone (90-10). Les fractions intéressantes sont concentrées à sec. Le résidu est dissous dans 20 ml d'éther éthylique. On ajoute 0,5 g de tert-butylamine. On concentre à sec et triture dans l'éther de pétrole. On observe une cristallisation. On essore, lave à l'éther de pétrole, sèche à 50 °C sous 133 Pa. On obtient 2,7 g de (Z),(R,S)-2-{4-[3-(6-hydroxy-2,5,7,8-tétraméthylchroman-2-yl)prop-2-én-1-yl]phénoxy}isobutyrate de tert-butylamine, P.F (cap) : 125-128°C Rendement : 63 %.

### Exemples 16-17

En opérant comme décrit dans l'exemple 15, ont été préparés les composés objet des exemples suivants :
16) l'acide (Z)-2-{4-[3-(3,5-di-tert-butyl-4-hydroxyphényl)prop-2-èn-1-yl]phénoxy}isobutyrique, P.F. (cap) : 111-114 °C (éther de pétrole).
17) le (Z),(R,S)-2-{4-[6-(6-hydroxy-2,5,7,8-tétraméthylchroman-2-yl)hex-5-én-1-yl]phénoxy} isobutyrate de sodium, (lyophilisat).

### Exemple 18

(R,S)-2-{4-[3-(6-hydroxy-2,5,7,8-tétraméthylchroman-2-yl)propyl]phénoxy} isobutyrate de tert-butylamine

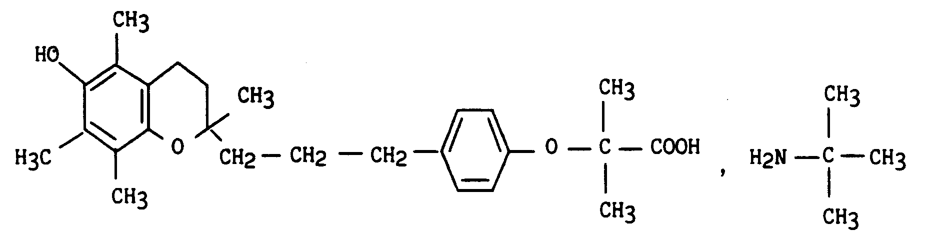

Dans un appareil à hydrogéner de Parr, on introduit 4,05 g (0,079 mole) de (Z),(R,S)-2-{4-[3-(6-éthoxyméthoxy-2,5,7,8-tétraméthylchroman-2-yl)prop-2-èn-1-yl]phénoxy}isobutyrate d'éthyle, 80 ml d'éthanol et 0,45 g de charbon palladié à 5 %. On hydrogène sous une pression de 5.10⁵ Pa, à 50 °C pendant 20 heures. On filtre et concentre à sec. Le résidu est chromatographié sur 170 cm³ de silice Amicon (0,035-0,070 mm) en éluant avec un mélange de toluène et d'acétate d'éthyle (96-4). Les fractions intéressantes sont concentrées à sec. On obtient 2,7 g de (R,S)-2-{4-[3-(6-éthoxyméthoxy-2,5,7,8-tétraméthylchroman-2-yl)-propyl]phénoxy} isobutyrate d'éthyle, sous forme de produit gommeux.
Dans un ballon muni d'une agitation et d'un réfrigérant, on introduit 6,0 g (0,012 mole) de (R,S)-2-{4-[3-(6-éthoxyméthoxy-2,5,7,8-tétra méthylchroman -2-yl)propyl]phénoxy} isobutyrate d'éthyle, précédemment obtenu, 100 ml d'éthanol et 20 ml d'HCl/N. On porte au reflux pendant 3 heures. On concentre à sec. On reprend le résidu dans l'éther éthylique, lave avec une solution saturée de chlorure de sodium, sèche sur sulfate de sodium et concentre à sec. Le résidu est repris dans 250 ml d'éthanol. On ajoute 25 ml de soude N et abandonne une nuit à température ambiante. On neutralise par 25 ml d'HCl/N et concentre à sec. Le résidu est repris dans l'éther éthylique, lavé avec une solution saturée de chlorure de sodium, séché sur sulfate de sodium et concentré à sec.

Le résidu est chromatographié sur 450 cm³ de silice Amicon (0,035-0,070 mm) en éluant avec un mélange de toluène et d'acétone (95-5). Les fractions intéressantes sont concentrées à une température inférieure à 45 °C. Le résidu est dissous dans 20 ml d'éther.

On ajoute 0,35 g de tert-butylamine. On dilue progressivement avec 80 ml d'éther de pétrole. On abandonne une nuit au réfrigérateur, essore les cristaux, lave à l'éther de pétrole et sèche à 40 °C sous 133 Pa. On obtient 1,4 g de (R,S)-2-{4-[3-(6-hydroxy-2,5,7,8-tétraméthylchroman-2-yl)propyl]phénoxy} isobutyrate de tert-butylamine, P.F (cap) : 115-118 °C. Rendement : 23 %.

### Exemples 19-22

En opérant comme décrit dans l'exemple 18, ont été préparés les composés objet des exemples suivants :
19) le (R,S)-2-{4-[6-(6-hydroxy-2,5,7,8-tétraméthylchroman-2-yl)hexyl] phénoxy}-2-méthylpropionate de sodium, (lyophilisat).
20) l'acide 2-{4-[3-(3,5-di-tert-butyl-4-hydroxyphényl)propyl]phénoxy} isobutyrique, P.F (cap) : 82-83 °C (éther éthylique).
21) le 2-{4-chloro-3-[2-(3,5-ditert-butyl-4-hydroxyphényl)propyl]phénoxy} isobutyrate de sodium (lyophilisat).
22) le (R,S) 2-{4-chloro-3-[3-(6-hydroxy-2,5,7,8-tétraméthyl-3,4-dihydrobenzopyran-2-yl)propyl]phénoxy} isobutyrate de sodium, (lyophilisat).

### Exemple 23

2-{4-[2-(4-hydroxy-3,5-ditert-butylphénylthio)éthoxy]phénoxy isobutyrate de tert-butylamine.

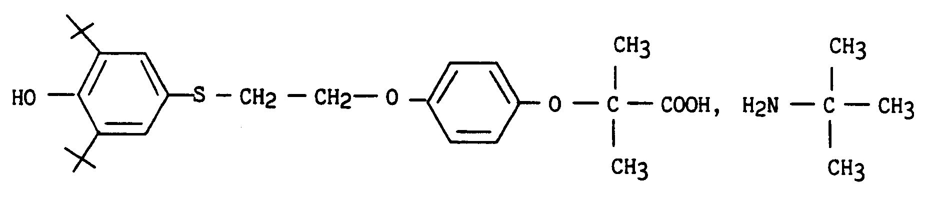

Dans un tricol muni d'une agitation, d'un réfrigérant et d'une ampoule à brome, on introduit 6,7 g (0,03 mole) de 2-(4-hydroxyphénoxy)isobutyrate d'éthyle, 10,2 g (0,03 mole) de 2-(4-éthoxyméthoxy-3,5-ditert-butyl phénylthio) éthanol, 7,9 g (0,03 mole) de triphénylphosphine et 160 ml de tétrahydrofurane.
On refroidit à 5 °C et coule en une heure 5,2 g d'azodicarboxylate de diéthyle dissous dans 40 ml de tétrahydrofurane.
On maintient l'agitation durant 7 heures à température ambiante, puis refroidit à 5 °C et ajoute à nouveau 7,9 g (0,03 mole) de triphénylphosphine puis 5,2 g (0,03 mole) d'azodicarboxylate de diéthyle.
On agite durant 16 heures à température ambiante puis concentre à sec à une température inférieure à 35 °C. On ajoute du cyclohexane et triture jusqu'à ce que l'on observe une cristallisation. On filtre et concentre à sec le filtrat.Le résidu est chromatographié sur 2 litres de silice Amicon/0,035-0,07 mm en éluant avec un mélange de dichlorométhane-cyclohéxane (70-30). Les fractions retenues sont concentrées à sec et on obtient 4,5 g de 2-{4-[2-(4-éthoxyméthoxy-3,5-ditert-butyl phénylthio)éthoxy]phénoxy}isobutyrate d'éthyle (Rendement : 27 %) sous forme de produit gommeux.
Dans un tricol de 250 ml, muni d'une agitation et d'un réfrigérant, on introduit 4,47 g de 2-{4-[2-(4-éthoxyméthoxy-3,5-ditert-butylphénylthio) éthoxy]phénoxy}isobutyrate d'éthyle précédemment obtenu, 70 ml d'éthanol et 11 ml de solution normale d'hydroxyde de sodium.
On porte à reflux pendant 16 heures puis refroidit, neutralise avec 11 ml d'HCl N et concentre à sec. On reprend le résidu dans l'éther, lave à l'eau, sèche sur sulfate de sodium et concentre à sec. On chromatographie le résidu sur 120 g de silice en éluant d'abord au dichlorométhane puis avec un mélange dichlorométhane-acétone (95-5). Les fractions retenues sont concentrées à sec. On obtient 4,0 g d'acide 2-{4-[2-(4-éthoxyméthoxy-3,5-ditert-butylphénylthio)éthoxy]phénoxy}isobutyrique, sous forme de produit gommeux (Rendement : 95 %).
Dans un ballon de 250 ml, on introduit 3,8 g (0,0073 mole) d'acide 2-{4-[2-(4-éthoxyméthoxy-3,5-ditert-butylphénylthio)éthoxy]phénoxy} isobutyrique, précédemment obtenu et 60 ml d'acide chlorhydrique 4 N dans le dioxane. On abandonne 20 heures à température ambiante. On fait passer un courant d'azote de façon à éliminer une partie de l'excès d'acide chlorhydrique. On concentre à sec sans chauffer puis reprend le résidu dans l'éther, lave plusieurs fois à l'eau, sèche sur sulfate de sodium et concentre à sec. Le résidu est chromatographié sur 300 ml de silice Amicon (0,035 - 0,07 mm) en éluant d'abord avec un mélange dichlorométhaneacétone (90-10). Les franctions retenues sont concentrées à sec. On obtient 2,9 g de produit gommeux que l'on dissout dans 30 ml de cyclohexane. On ajoute 0,45 g de tert-butylamine dissous dans 20 ml de cyclohexane, sèche à 50 °C sous 0,5 torr et obtient 2,8 g de 2-{4-[2-(4-hydroxy-3,5-ditert-butylphénylthio)éthoxy]phénoxy}isobutyrate de tert-butylamine fondant (cap) à 157-159 °C (Rendement : 72 %)

### Exemple 24

2-{4-[2-(3,5-ditert-butyl-4-hydroxyphénylthio]-1-oxoéthylphénoxy} isobutyrate d'éthyle :

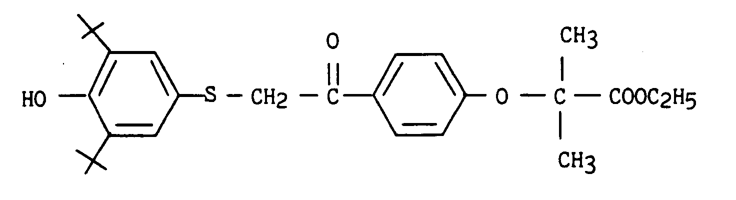

Dans un tricol muni d'une agitation et d'un réfrigérant, on introduit 9,6 g (0,04 mole) de 4-hydroxy-3,5-ditert-butyl phénylthiol, 13,2 g de 2-(4-bromoacétylphénoxy) isobutyrate d'éthyle, 5,6 g de carbonate de potassium et 180 ml d'acétone. On agite 3 jours à température ambiante. On filtre et concentre à sec. Le résidu est repris dans le dichlorométhane. On lave la solution avec une solution aqueuse de bicarbonate de sodium à 10 %. On sèche sur sulfate de sodium et concentre à sec. Le résidu est chromatographié sur 2 l de silice Amicon (0,035-0,070 mm) en éluant avec un mélange dichlorométhane/cyclohexane (50/50). Les fractions retenues sont concentrées à sec. On obtient 15,74 g de 2-{4-[1-oxo-2-(4-hydroxy-3,5-ditert-butyl phénylthio)éthyl]phénoxy} isobutyrate d'éthyle sous forme de gomme non cristallisée (Rendement : 81 %).

Le 2-(4-bromoacétylphénoxy) isobutyrate d'éthyle, utilisé comme matière première a été préparé comme suit :
Dans un tricol muni d'une agitation, on introduit 10 g (0,04 mole) de 2-(4-acétylphénoxy)isobutyrate d'éthyle et 100 ml de tétrahydrofurane. On ajoute en 1 heure 19,9 g d'hydrotribromure de pyrrolidone en solution dans 400 ml de tétrahydrofurane à température inférieure à 25 °C. On agite 20 heures à température ambiante. On filtre et concentre à sec sans dépasser 40 °C. On reprend le résidu dans le dichlorométhane, lave avec une solution à 10 % de bicarbonate de sodium, sèche sur sulfate de sodium et concentre à sec. Le résidu est chromatographié sur 1,2 l de silice Amicon (0,035-0,070 mm) en éluant avec un mélange dichlorométhane-cyclohexane (80/20). Les fractions retenues sont concentrées à sec. On obtient 7,85 g de 4-(2-bromoacétylphénoxy)isobutyrate d'éthyle sous forme de liquide épais (Rendement : 60 %).

Le 2-(4-acétyl phénoxy)isobutyrate d'éthyle de départ a lui-même été préparé comme suit :
Dans un tricol muni d'une agitation et d'un réfrigérant, on introduit 54,4 g de 4-hydroxy acétophénone, 375 ml de 2-bromoisobutyrate d'éthyle, 175 g de carbonate de potassium, 1,2 l de méthylisobutyl cétone et 3 g d'iodure de potassium. On porte au reflux 20 heures, puis refroidit, filtre et concentre à sec. On chromatographie le résidu sur 4 l de silice Amicon (0,035-0,070 mm) en éluant avec du dichlorométhane. Les fractions retenues sont concentrées à sec. On obtient 100g de 2-(4-acétylphénoxy)isobutyrate d'éthyle sous forme de liquide épais (Rendement quantitatif).

### Exemple 25

En opérant par analogie avec l'exemple 24, a été préparé le composé suivant :
le 2-{4-[2-(3,5-ditert-butyl-4-hydroxyphényl)-1-oxoéthyl]phénoxy} isobutyrate d'éthyle, PF (K) : 117 °C (cyclohexane).

### Exemple 26

L'acide 2-{4-[2-(3,5-ditert-butyl-4-hydroxyphénylthio)-1-oxoéthyl]phénoxy} isobutyrique :

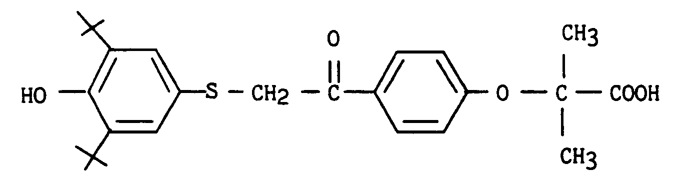

Dans un ballon muni d'une agitation magnétique, on introduit 4,86 g (0,01 mole) de 2-{4-[1-oxo-2-(4-hydroxy-3,5-ditert-butylphénylthio)éthyl] phénoxy} isobutyrate d'éthyle, 70 ml d'éthanol et 12 ml de solution normale de soude. On porte à reflux 1 heure 30, puis refroidit, neutralise par 12 ml de solution normale d'acide chlorhydrique et concentre à sec. Le résidu est repris dans l'éther. On lave à l'eau, sèche sur sulfate de sodium et concentre à sec. Le résidu est chromatographié sur 300 ml de silice Amicon (0,035-0,070 mm) en éluant d'abord avec du dichlorométhane pur puis avec un mélange dichlorométhane-méthanol (97-3).
Les fractions retenues sont concentrées à sec. Le résidu est trituré dans l'éther de pétrole et séché à température inférieure à 40 °C. On obtient 1,5 g de l'acide 2-{4-[2-(3,5-ditert-butyl-4-hydroxyphénoxythio)éthyl] phénoxy} isobutyrique, PF (K) : 141 °C (Rendement : 33 %).

### Exemple 27

En opérant par analogie avec l'exemple 26, a été préparé le composé suivant :
le 2-{4-[2-(3,5-ditert-butyl-4-hydroxyphénoxy)-1-oxoéthyl]phénoxy} isobutyrate de tert-butylamine, PF (cap) : 162-165 °C.

### Exemple 28

Le (R,S)-2-{4-[2-(3,5-ditert-butyl-4-hydroxyphénylthio)-1-hydroxyéthyl] phénoxy} isobutyrate de tert-butylamine :

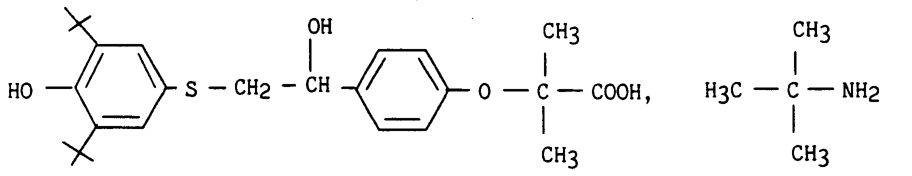

Dans un tricol de 250 ml muni d'une agitation et d'une ampoule de coulée, on introduit 4,86 g (0,01 mole) de 2-{4-[1-oxo-2-(4-hydroxy-3,5-ditert-butyl phénylthio)éthyl] phénoxy} isobutyrate d'éthyle et 50 ml de tétrahydrofurane. On ajoute à 5 °C, 0,5 g de borohydrure de sodium puis 3,5 ml d'eau. On agite 1 heure à température ambiante. On refroidit à 5 °C et ajoute à nouveau 0,5 g de borohydrure de sodium. On agite 4 heures à température ambiante, puis concentre à sec à température inférieure à 40 °C. On reprend dans le dichlorométhane et lave avec une solution à 10 % de bicarbonate de sodium. On sèche sur sulfate de sodium et concentre à sec. Le résidu est chromatographié sur 400 ml de silice Amicon (0,035-0,070 mm) en éluant avec du dichlorométhane. Les fractions retenues sont concentrées à sec et l'on obtient 4,2 g de (R,S)-2-{4-[1-hydroxy-2-(4-hydroxy-3,5-ditert-butylphénylthio)éthyl] phénoxy} isobutyrate d'éthyle (Rendement : 86 %) sous forme de produit gommeux.
Ce produit est repris dans 84 ml d'éthanol. On ajoute 10 ml de soude N et abandonne 72 heures à température ambiante. On ajoute 11 ml d'acide chlorhydrique N et on concentre à sec à température inférieure à 35 °C. Le résidu est repris dans l'éther éthylique. On lave à l'eau, sèche sur sulfate de sodium et concentre à sec. Le résidu est chromatographié sur silice Amicon (0,035-0,070 mm) en éluant d'abord au dichlorométhane puis avec un mélange dichlorométhane-acétone (97-3). Les fractions retenues sont concentrées à sec. Le résidu est repris dans l'eau. On ajoute 0,35 g de tert-butylamine. La solution aqueuse est extraite à l'éther de pétrole, puis est lyophilisée. On obtient 1,8 g de (R,S)-2-{4-[1-hydroxy-2-(4-hydroxy-3,5-ditert-butyl phénylthio)éthyl]phénoxy} isobutyrate de tert-butyl amine sous forme de lyophilisat (Rendement :39 %).

### Exemple 29

L'acide 2-{4-[2-(4-hydroxy-3,5-ditert-butylphénoxy)éthoxy]phénoxy} isobutyrique :

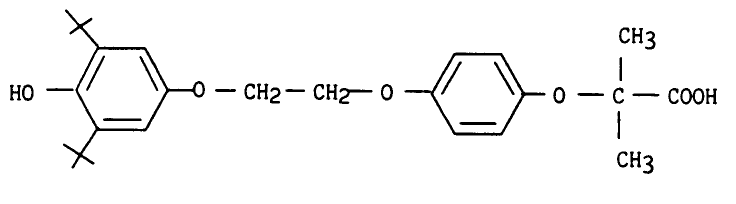

Dans un tricol muni d'une agitation, d'un régrigérant et d'une ampoule de coulée, on introduit 6,6 g (0,0293 mole) de 2-(4-hydroxyphénoxy) isobutyrate d'éthyle, 7,7 g de triphénylphosphine et 160 ml de tétrahydrofurane. On refroidit à 5 °C et on coule 5,1 g d'azadicarboxylate de diéthyle dissous dans 40 ml de tétrahydrofurane à température inférieure à 10 °C. On agite 1 heure à 5 °C. On ajoute 9,5 g de 2-(4-éthoxyméthoxy-3,5-ditert-butylphénoxy)éthanol dissous dans 50 ml de tétrahydrofurane. On agite 1 heure à 5 °C puis 20 heures à température ambiante. On concentre à sec à température inférieure à 30 °C. Le résidu est trituré dans le cyclohexane et filtré. Le filtrat est concentré à sec puis chromatographié sur 2,9 l de silice Amicon (0,035-0,070 mm) en éluant avec un mélange dichlorométhane-cyclohexane (80-20). Les fractions retenues sont concentrées à sec. Le 2-{4-[2-(4-éthoxyméthoxy-3,5-ditert-butylphénoxy)éthoxy]phénoxy} isobutyrate d'éthyle, cristallise, PF (K) : 74 °C (Rendement : 31 %).

Dans un tricol de 250 ml, muni d'une agitation et d'un réfrigérant, on introduit 4,8 g de 2-{4-[2-(4-éthoxyméthoxy-3,5-ditert-butyl phénoxy) éthoxy]phénoxy}isobutyrate d'éthyle obtenu précédemment, 70 ml d'éthanol et 12 ml de soude N. On porte au reflux 16 heures, on refroidit, neutralise par 12,2 ml d'HClN, puis concentre à sec. On reprend le résidu dans l'éther éthylique, lave à l'eau, sèche sur sulfate de sodium et concentre à sec. Le résidu est chromatograhié sur 300 ml de silice Amicon (0,035-0,070 mm) en éluant d'abord au dichlorométhane pur puis avec un mélange dichlorométhane-acétone (85-15). Les fractions retenues sont concentrées à sec. On obtient 4,25 g d'acide 2-{4-[2-(4-éthoxyméthoxy-3,5-ditert-butyl phénoxy)éthoxy]phénoxy} isobutyrique sous forme de gomme (Rendement : 94 %).

Dans un ballon de 250 ml, on introduit 4 g de l'acide ci-dessus obtenu et 60 ml d'acide chlorhydrique 4N dans le dioxane. On abandonne 24 heures à température ambiante. On fait passer un courant d'azote de façon à éliminer une partie de l'excès d'acide chlorhydrique. On concentre à sec à température inférieure à 35 °C. Le résidu est repris dans l'éther éthylique, lavé à l'eau, séché sur sulfate de sodium et concentré à sec à température inférieure à 35°C. Le résidu est chromatographié sur 120 g de silice Amicon (0,035-0,070 mm) en éluant d'abord au dichlorométhane pur puis avec un mélange dichlorométhane-acétone (90-10). Les fractions retenues sont concentrées à sec. On obtient 2,85 g d'acide 2-{4-[2-(4-hydroxy-3,5-ditert-butyl phénoxy)éthoxy]phénoxy} isobutyrique qui fond à 124 °C (Rendement : 81 %).

### Exemple 30

(R,S)-2-{4-[1-chloro-2-(3,5-ditert-butyl-4-hydroxy phénoxy)éthyl] phénoxy} isobutyrate d'éthyle.

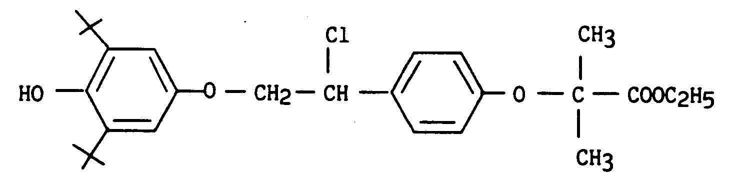

Dans un tricol muni d'une agitation, on introduit 5,8 g (0,011 mole) de 2-{4-[1-oxo-2-(4-éthoxyméthoxy-3,5-ditert-butyl phénoxy)éthyl] phénoxy} isobutyrate d'éthyle et 58 ml de tétrahydrofurane. On refroidit à 5 °C puis on ajoute 0,417 g de borohydrure de sodium et 3 ml d'eau. On agite 2 heures à 5 °C puis 20 heures à température ambiante. On concentre à sec à température inférieure à 30 °C. Le résidu est repris dans le dichlorométhane, lavé avec une solution à 10 % de bicarbonate de sodium, séché sur sulfate de sodium et concentré à sec. Le résidu est chromatographié sur 450 ml de silice Amicon (0,035-0,070 mm) en éluant d'abord avec du dichlorométhane pur puis avec un mélange dichlorométhaneacétate d'éthyle. Les fractions retenues sont concentrées à sec. On obtient 3,6 g de (R,S)-2-{4-[1-hydroxy-2-(4-hydroxy-3,5-ditert-butyl phénoxy)éthyl] phénoxy} isobutyrate d'éthyle sous forme de gomme (Rendement : 62 %).

Dans un ballon de 500 ml, on introduit 6,2 g de l'hydroxyester préparé précédemment (0,0117 mole) et 100 ml d'acide chlorhydrique 4N dans le dioxane. On abandonne 24 heures à température ambiante. On fait passer un courant d'azote de façon à éliminer une partie de l'excès d'acide chlorhydrique puis on concentre à sec à température inférieure à 30 °C. Le résidu est repris dans l'éther, lavé avec une solution saturée de chlorure de sodium, lavé à l'eau, séché sur sulfate de sodium et concentré à sec. Le résidu est chromatographié sur 450 ml de silice en éluant avec un mélange de dichlorométhane-cyclohexane. Les fractions retenues sont concentrées à sec et le résidu est trituré dans l'éther de pétrole. On essore les cristaux et sèche à 50 °C sous 0,5 torr. On obtient 2,5 g de (R,S)-2-{4-[1-chloro-2-(4-hydroxy-3,5-ditert-butyl phénoxy) éthyl] phénoxy} isobutyrate d'éthyle, PF (cap) : 80-81 °C (Rendement = 43 %).

### Exemple 31

### ETUDE PHARMACOLOGIQUE

L'action des dérivés de la présente invention a été démontrée sur des LDL humaines et animales. L'activité inhibitrice des composés vis à vis de la modification oxydative des LDL, induite par le sulfate de cuivre et par des cellules endothéliales d'aorte de lapin, a été demontrée tant in vitro (sur les LDL humaines) qu'après administration par voie orale chez le lapin Watanabe. L'activité des composés a été testée de façon comparative au probucol et à la vitamine E pris comme produits de référence.

### 1. ETUDE IN VITRO

### 1.1 Matériel et méthodes

### 1.1.1 Modification des LDL par le sulfate de cuivre

Les LDL humaines sont incubées 24 heures en présence de sulfate de cuivre (5.10⁻⁶ M) et en absence ou en présence des composés testés (10⁻⁹ M à 10⁻⁴ M).

Après incubation, la peroxydation des LDL est évaluée par électrophorèse sur gel d'agar et par la formation de l'un des produits de la peroxydation lipidique : le malondialdéhyde (MDA) (Parthasarathy S., Young S.G., Witztum J.L., Pittman R.C., et Steinberg D.; J. Clin. Invest. 77 ; 641-644, 1986).
L'activité des composés testés est évaluée par le calcul des concentrations réduisant de 50 % (IC50) la production de MDA par rapport aux expériences contrôles en absence de produit.

### 1.1.2 Modification des LDL par les cellules endothéliales

Les LDL humaines sont incubées 24 heures en présence de cellules endothéliales d'aorte de lapin (lignée RECL B4 fournie par le Professeur Steinberg, USA), et en absence ou en présence des composés testés (10-9 M à 10-4 M).
Après incubation, la peroxydation des LDL est évaluée par électrophorèse sur gel d'agar et par la formation de l'un des produits de la peroxydation lipidique : le malondialdéhyde (MDA) (Steinbrecher U.P., Parthasarathy S, Leake D.S., Witztum J.L., et Steinberg D.; Proc. Nat. Acad. Sci. USA 81. 3883-3887, 1984).
L'activité des composés testés est évaluée par le calcul des concentrations réduisant de 50 % (IC50) la production de MDA par rapport aux expériences contrôles en absence de produit.

### 1.2 Résultats

### 1.2.1 Effet sur la modification des LDL

Le tableau A rassemble les IC50, appréciant le pouvoir inhibiteur de la peroxydation lipidique des LDL humaines, obtenues avec un échantillon des composés de l'invention et les produits de référence : probucol et vitamine E, sur les deux tests d'incubation d'oxydation des LDL : par le sulfate de cuivre (Cu²⁺) ou par les cellules endothéliales (EC).

**TABLEAU A**

| **COMPOSES** | **IC50 (M)** | |
|---|---|---|
| | Cu²⁺ | EC |
| Exemple 1 | 5.10⁻⁸ | 3.10⁻⁹ |
| Exemple 3 | 9.10⁻⁸ | |
| Exemple 4 | 3.10⁻⁸ | |
| Exemple 5 | 4.10⁻⁸ | |
| Exemple 9 | 5.10⁻⁷ | |
| Exemple 10 | 2.10⁻⁸ | 7.10⁻⁹ |
| Exemple 15 | 4.10⁻⁸ | |
| Exemple 18 | 6.10⁻⁸ | |
| PROBUCOL | 3.10⁻⁶ | 4.10⁻⁶ |
| VITAMINE E | >10⁻⁴ | 4.10⁻⁶ |

Ces résultats indiquent clairement la plus grande puissance notamment des composés des exemples 1, 3, 4 et 5 par rapport au probucol ou à la vitamine E à protéger les LDL humaines vis à vis des modifications induites par le sulfate de cuivre et les cellules endothéliales.
Ces composés, dont les IC50 vis à vis de la peroxydation induite par le sulfate de cuivre (5.10⁻⁶ M) se situent entre 2.10⁻⁸ et 5.10⁻⁷, sont 100 fois plus puissants que le probucol sur ce test ; concernant le test utilisant les cellules endothéliales, le dérivé concernant l'exemple 1, avec une IC50 de 3.10⁻⁹ M, s'avère 1000 fois plus puissant que le probucol.

A titre d'exemple, les effets du composé de l'exemple 1 ont été illustré par les figures 1 et 2 ci-après :

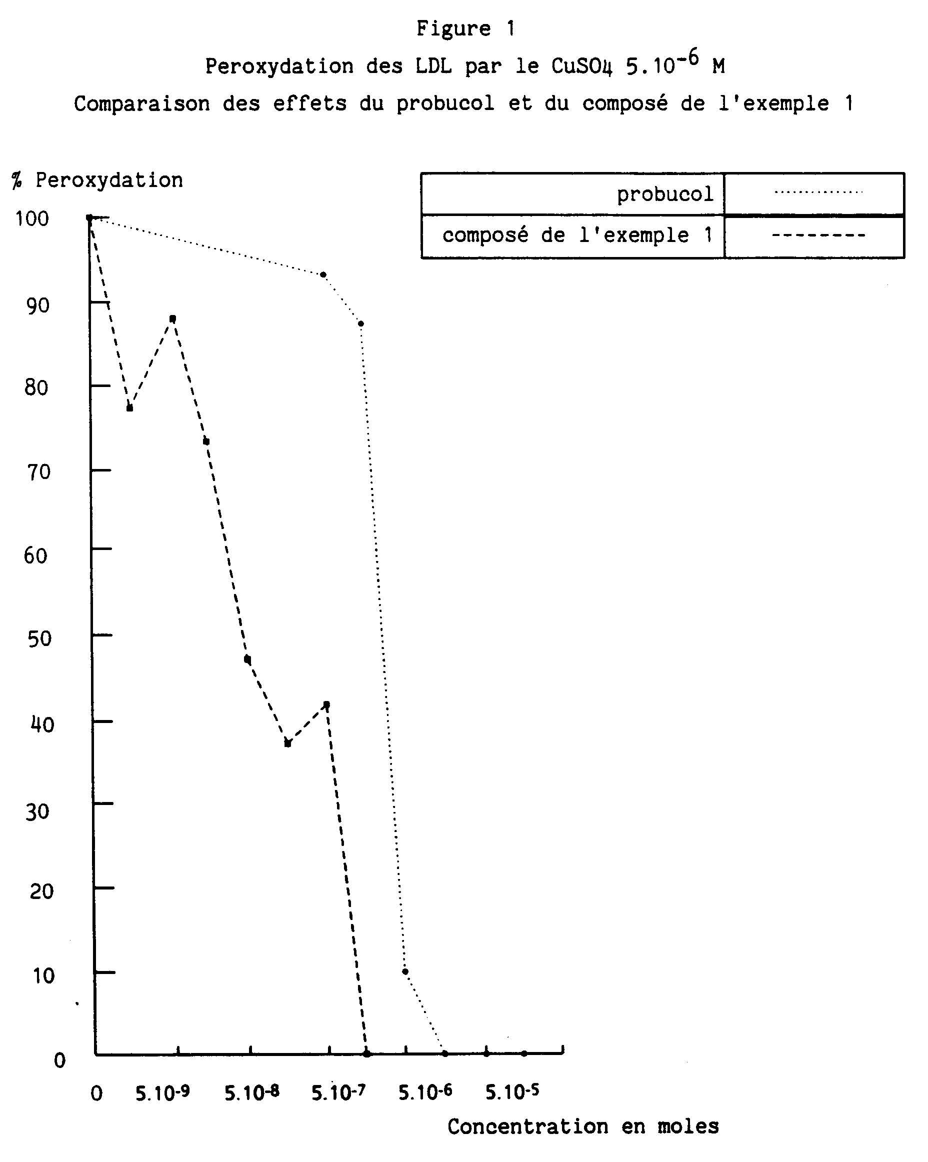

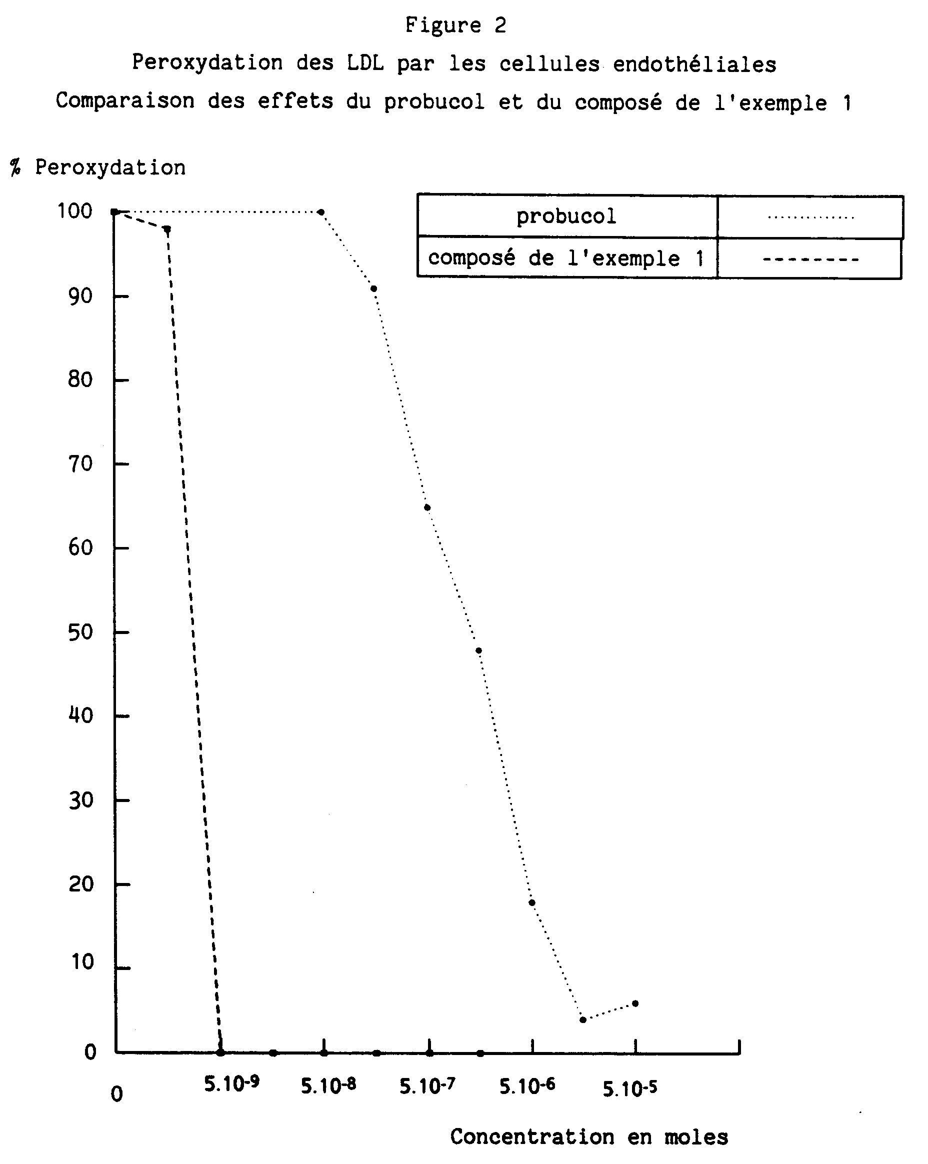

### 2. ETUDE EX VIVO

### 2.1 Matériels et méthodes

Des lapins Watanabe (lapins génétiquement hyperlipidémiques), de poids variant de 3 kg à 5 kg, sont utilisés. Les animaux sont traités par voie orale soit par le véhicule des composés testés (groupe contrôle) soit par les produits testés à la dose de 50 mg/kg/jour pendant 3 jours.

En fin de traitement, les LDL de ces animaux sont préparées par ultracentrifugation et soumises à une oxydation par le sulfate de cuivre (5.10⁻⁶ M). La peroxydation lipidique des LDL est appréciée, après différents temps d'incubation avec le sulfate de cuivre (de 2 à 24 heures) par la mesure de la formation de MDA.

### 2.2 Résultats

Le tableau B rassemble les valeurs de production de MDA selon le temps d'incubation avec le sulfate de cuivre des LDL provenant des animaux contrôles et traités par le composé de l'exemple 1 à différentes doses ou le probucol.

**TABLEAU B**

| Production de MDA (nmol/mg protéines) | | | | | | |
|---|---|---|---|---|---|---|
| **COMPOSES** | **NOMBRE D'ANIMAUX** | Temps d'incubation des LDL avec le sulfate de cuivre (heures) | | | | |
| | | 2 | 4 | 6 | 8 | 24 |
| Contrôle | 6 | 3,31 ± 0,41 | 4,84 ± 0,59 | 13,41 ± 2,29 | 26,04 ± 2,82 | 28,06 ± 2,87 |
| Exemple 1 50 mg/kg/j | 4 | 0,66 ± 0,31 | 0,35 ± 0,35 | 0,31 ± 0,18 | 0,26 ± 0,26 | 0,86 ± 0,32 |
| Probucol 250 mg/kg/j | 5 | 1,85 ± 0,27 | 3,50 ± 0,27 | 4,90 ± 0,89 | 12,26 ± 2,45 | 23,81 ± 0,37 |

D'après ces résultats, également illustrés par la figure 3, la peroxydation des LDL des lapins Watanabe traités par le probucol à la dose de 250 mg/kg/j est retardée d'environ 2 heures par rapport à celle des animaux contrôles.

Le composé de l'exemple 1, dans les mêmes conditions expérimentales, inhibe complètement la peroxydation des LDL induite par le sulfate de cuivre dès la dose de 50 mg/kg/jour.

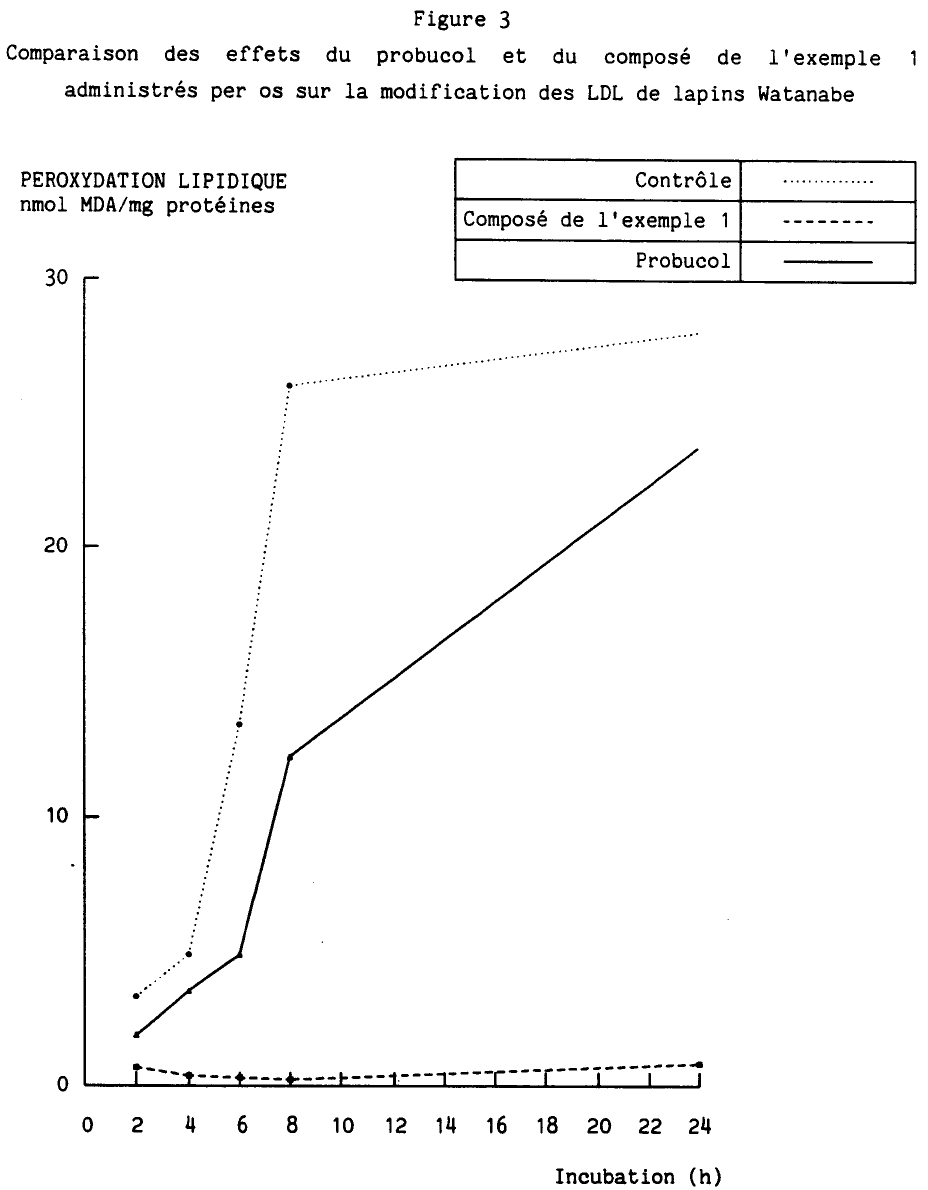

### 3. CONCLUSION

Les résultats rapportés démontrent d'une part que les composés de l'invention protègent les LDL humaines in vitro vis à vis des modifications oxydatives de manière beaucoup plus puissante que le probucol et la vitamine E, d'autre part qu'après administration chez l'animal, par voie orale, ces composés provoquent une protection des LDL très nettement supérieure et de plus longue durée d'action par rapport au probucol.

## Revendications

1. Les acides et esters phénoxy isobutyriques substitués de formule I : 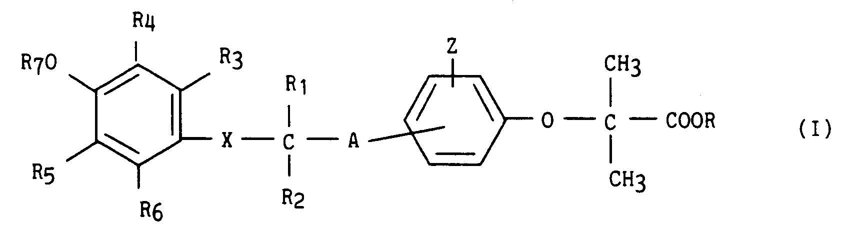 dans laquelle :
- X représente un atome d'oxygène, un atome de soufre ou une liaison simple ;
- A représente une liaison simple ou un radical hydrocarboné contenant de 1 à 9 atomes de carbone en chaîne droite ou ramifiée renfermant éventuellement une double liaison, un radical cyclopropyle, un atome d'oxygène ou un radical carbonyle, ou éventuellement substitué par un atome d'halogène ou un radical hydroxy ;
- R représente un atome d'hydrogène ou un radical alkyle de 1 à 6 atomes de carbone en chaîne droite ou ramifiée éventuellement s3bstitué par un ou deux radicaux hydroxy ;
- R₁ et R₃ :
. représentent chacun simultanément un atome d'hydrogène, ou
. forment ensemble un pont (CH₂)ₙ dans lequel n prend les valeurs 1 ou 2, ou
. R₁ représente :
. un radical méthyle, ou
. une liaison simple formant une double liaison avec le groupe A lorsque celui-ci est un radical hydrocarboné, et
. dans chacun de ces cas, simultanément R₃ représente un atome d'hydrogène ;
- R₂ et R₆, identiques ou différents, représentent chacun un atome d'hydrogène ou un radical méthyle ;
- R₄ et R₅, identiques ou différents, représentent chacun un radical alkyle de 1 à 6 atomes de carbone en chaîne droite ou ramifiée ;
- R₇ représente un atome d'hydrogène ou un groupement protecteur labile tel que par exemple un radical CH₃CO-, C₂H₅O-CH₂- ou benzyle ; et
- Z représente un atome d'hydrogène ou d'halogène ou un radical alkyle ou alkoxy contenant chacun de 1 à 5 atomes de carbone en chaîne droite ou ramifiée ;
et, quand ils existent, les énantiomères et diastéréoisomères correspondants,
ainsi que les sels physiologiquement tolérables des composés I avec des bases appropriées.

2. L'acide 2-{4-[2-(3,5-di-tert-butyl-4-hydroxyphénylthio)éthyl]phénoxy} isobutyrique.

3. L'acide 2-{4-[2-(4-hydroxy-2,3,5-triméthylphénylthio)éthyl]phénoxy} isobutyrique.

4. L'acide 2-[4-(3,5-di-tert-butyl-4-hydroxyphénylthiométhyl)phénoxy] isobutyrique.

5. Le sel de tert-butylamine de l'acide 2-{4-[3-(3,5-di-tert-butyl-4-hydroxyphénylthio)propyl]phénoxy}isobutyrique.

6. L'acide 2-{4-[2-(4-hydroxy-2,3,5-triméthylphénoxy)éthyl]phénoxy} isobutyrique.

7. L'acide 2-{4-[2-(3,5-di-tert-butyl-4-hydroxyphénoxy)éthyl]phénoxy} isobutyrique.

8. Le (Z),(R,S)-2-{4-[3-(6-hydroxy-2,5,7,8-tétraméthylchroman-2-yl)prop-2-én-1-yl]phénoxy}isobutyrate de tert-butylamine.

9. Le (R,S)-2-{4-[3-(6-hydroxy-2,5,7,8-tétraméthylchroman-2-yl)propyl] phénoxy}isobutyrate de tert-butylamine.

10. Le procédé de préparation des composés de la revendication 1 caractérisé en ce que l'on fait réagir :
a)
. soit un composé de formule IIa : 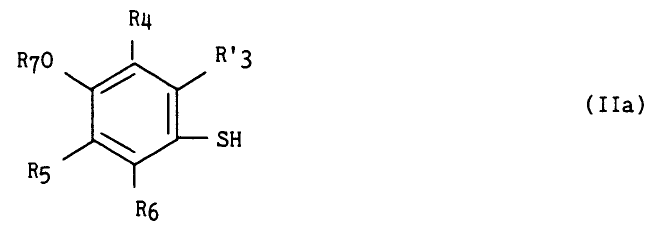 dans laquelle :
- R₄, R₅, R₆ et R₇ ont les significations définies dans la revendication 1, et
- R'₃ représente un atome d'hydrogène
. avec un composé de formule IIIa : 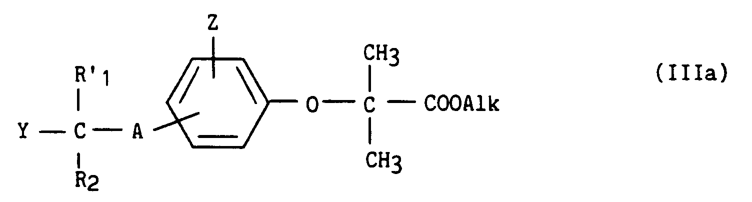 dans laquelle :
- R₂, A et Z ont les significations définies dans la revendication 1 ;
- R'₁ représente un atome d'hydrogène ou un radical méthyle ;
- Alk représente un radical alkyle de 1 à 6 atomes de carbone en chaine droite ou ramifiée,
et
- Y représente un atome de chlore ou de brome ;
. pour obtenir un composé de formule Ia₁ : 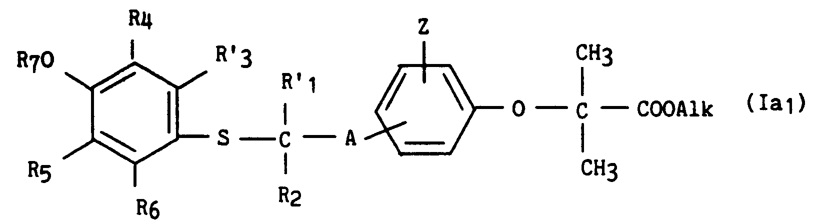 dans laquelle R'₁, R₂, R'₃, R₄, R₅, R₆, R₇, A, Z et Alk ont les significations précédemment définies,
lequel composé Ia₁ est, selon la nature de R₇, transformé, par des méthodes séquentielles de saponification, hydrolyse ou hydrogénolyse, en un composé de formule Ia₂ : 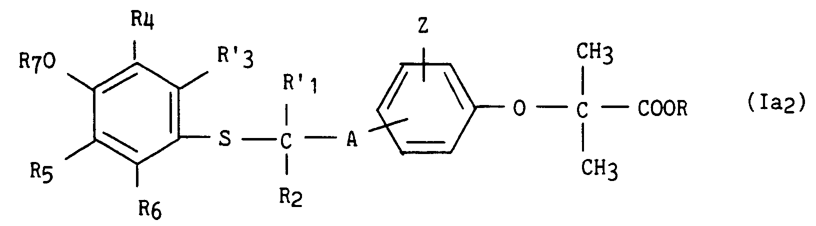 dans laquelle R'₁, R₂, R'₃, R₄, R₅, R₆, R₇, A, Z et R ont les significations définies dans la revendication 1,
b)
. soit un composé de formule IIb : 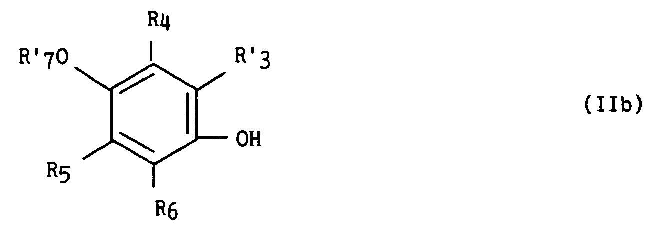 dans laquelle :
- R'₃, R₄, R₅ et R₆ ont les significations précédemment définies et
- R'₇ représente un groupement protecteur labile tel que : CH₃CO-, C₂H₅-O-CH₂- ou benzyle ;
. avec un composé de formule IIIb : 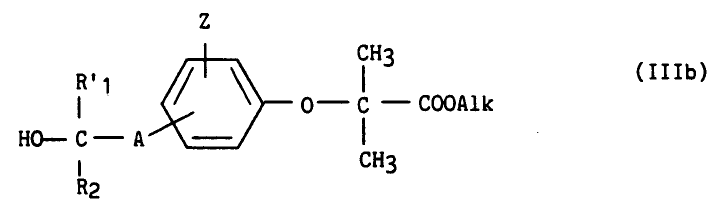 dans laquelle R'₁, R₂, A, Z et Alk ont les significations précédemment définies,
. pour obtenir un composé de formule Ib₁ : 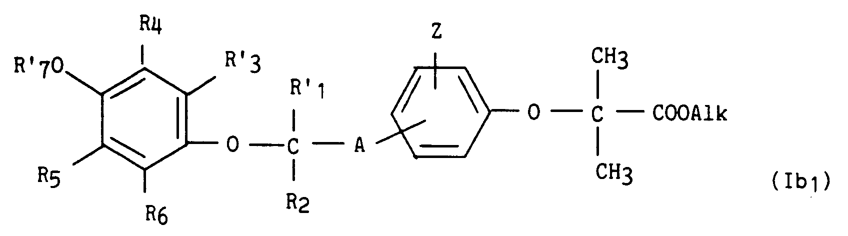 dans laquelle R'₁, R₂, R'₃, R₄, R₅, R₆, R'₇, A, Z et Alk ont les significations précédemment définies,
. lequel composé de formule Ib₁ est, selon la nature de R'₇, transformé par des méthodes séquentielles de saponification, hydrolyse ou hydrogénolyse en un composé de formule Ib₂ : 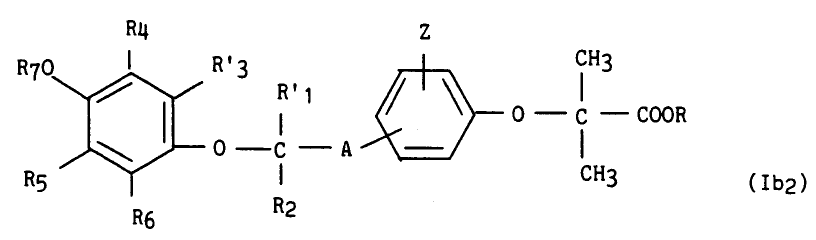 dans laquelle R'₁, R₂, R'₃, R₄, R₅, R₆, R₇, A, Z et R ont les significations précédemment définies ;
c)
. soit un composé de formule IIc : 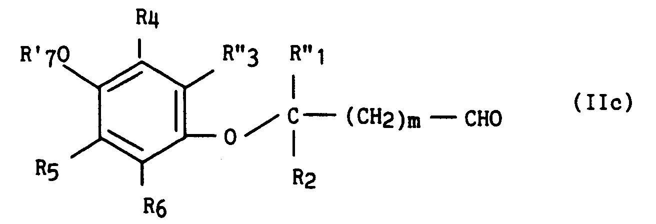 dans laquelle :
- R₂, R₄, R₅, R₆ et R'₇ ont les significations précédemment définies,
- R''₁ et R''₃ représentent ensemble un pont (CH₂)ₙ dans lequel n prend la signification définie dans la revendication 1, et
- m représente zéro ou un nombre entier de 1 à 5 ;
. avec un composé de formule IIIc : 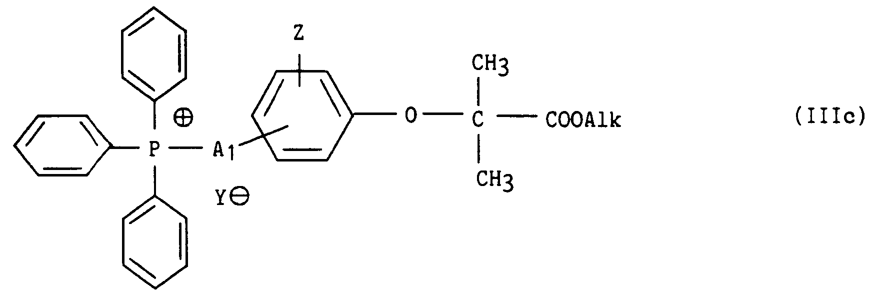 dans laquelle :
- Alk et Z et Y ont les significations précédemment définies,
- A₁ représente une liaison simple où un radical hydrocarboné contenant de 1 à 3 atomes de carbone en chaîne droite ou ramifiée ;
. pour obtenir un composé de formule Ic₁ : 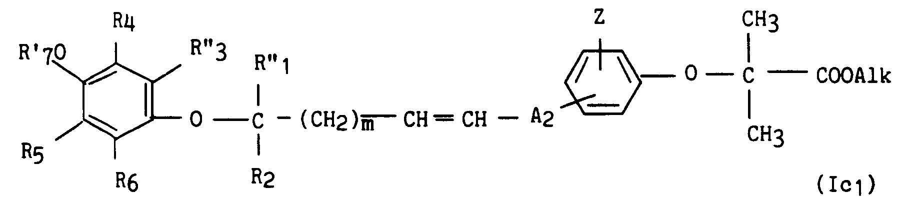 dans laquelle :
- R''₁, R₂, R''₃, R₄, R₅, R₆, R'₇, m, Z et Alk ont les significations précédemment définies, et
- A₂ représente une liaison simple ou un radical hydrocarboné contenant 1 ou 2 atomes de carbone en chaîne droite ou ramifiée ;
lequel composé de formule Ic₁ est réduit pour obtenir le composé de formule Ic₂ : 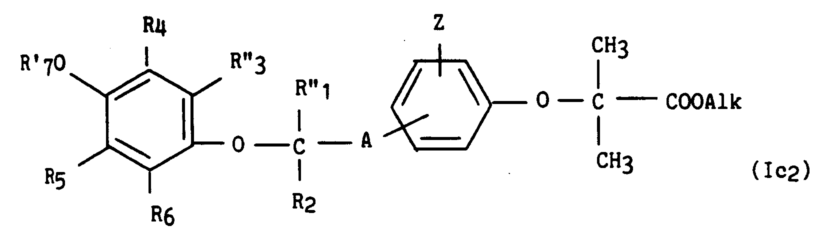 dans laquelle R''₁, R₂, R''₃, R₄, R₅, R₆, R'₇, A, Z et Alk ont les significations précédemment définies ;
lequel composé de formule Ic₂ est, selon la nature de R'₇, transformé par des méthodes séquentielles de saponification, hydrolyse ou hydrogénolyse en un composé de formule Ic₃ : 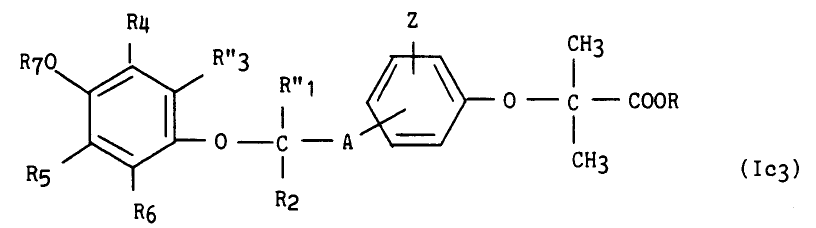 dans laquelle R''₁, R₂, R''₃, R₄, R₅, R₆, R'₇, A, Z et R ont les significations précédemment définies.
d) soit un composé de formule II d) : 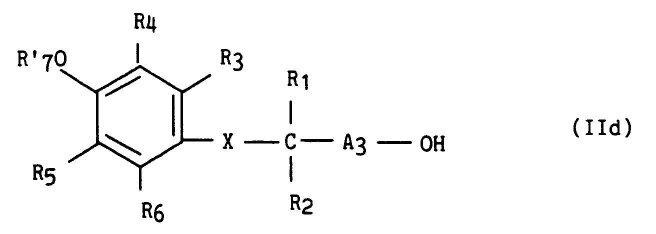 dans laquelle :
- R₁, R₂, R₃, R₄, R₅, R₆, R'₇ et X ont les significations précédemment définies, et
- A₃ représente une liaison simple ou un radical hydrocarboné contenant de 1 à 9 atomes de carbone en chaîne droite ou ramifiée,
avec un composé de formule IIId : 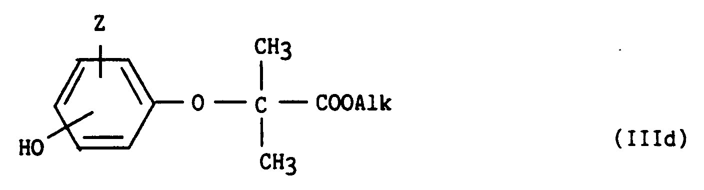 dans laquelle Z et Alk ont les significations précédemment définies,
pour obtenir un composé de formule Id₁ : 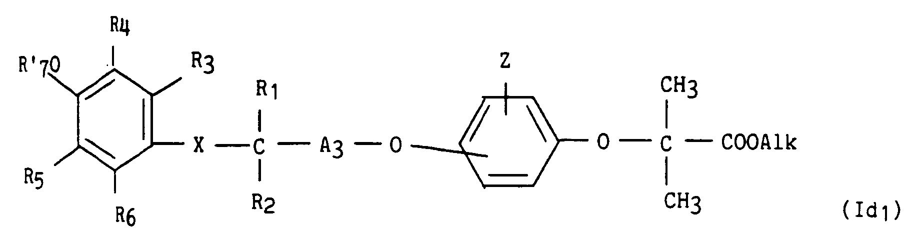 dans laquelle R₁, R₂, R₃, R₄, R₅, R₆, R'₇, X, A₃ et Alk ont les significations précédemment définies;
lequel composé de formule Id₁ ci-dessus défini peut être saponifié, hydrogénolysé ou hydrolysé pour donner l'acide correspondant de formule Id₂ : 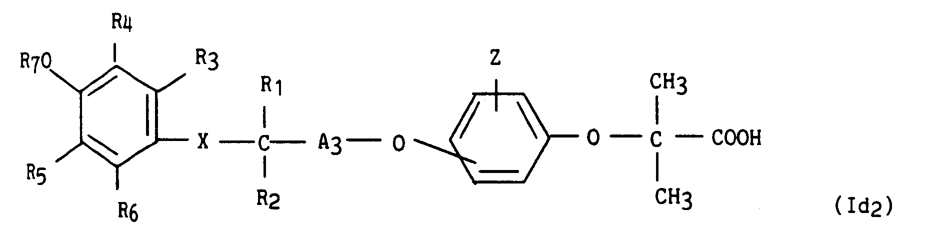 dans laquelle R₁, R₂, R₃, R₄, R₅, R₆, R₇, X, A₃ et Z ont les significations précédemment définies ;
e) soit un composé de formule IIe : 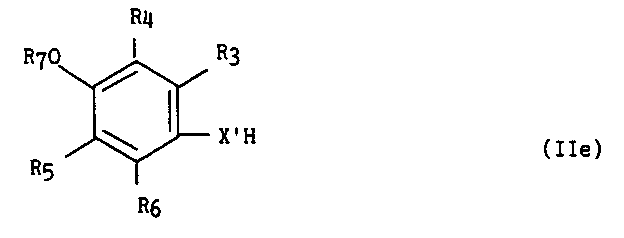 dans laquelle :
- R₃, R₄, R₅, R₆ et R₇ ont les significations précédemment définies, et
- X' représente un atome d'oxygène ou de soufre,
avec un composé de formule IIIe : 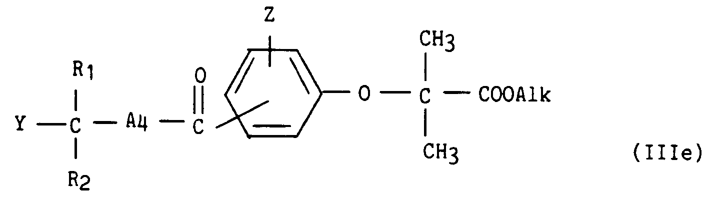 dans laquelle :
- Y, R₁, R₂, Z et Alk ont les significations précédemment définies, et
- A₄ représente une liaison simple ou une chaîne hydrocarbonée ayant de 1 à 8 atomes de carbone ;
pour obtenir un composé de formule Ie₁ : 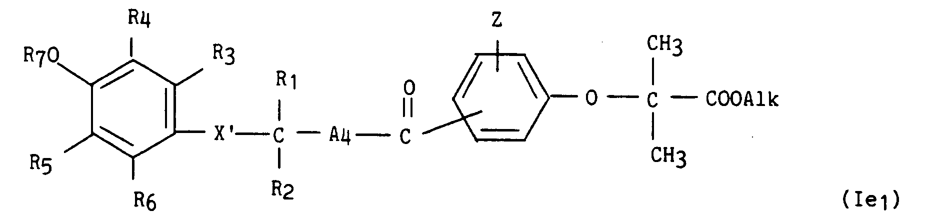 dans laquelle R₁, R₂, R₃, R₄, R₅, R₆, R₇, X', A₄, Z et Alk ont les significations précédemment définies ;
lequel composé de formule Ie₁ ci-dessus défini peut être saponifié pour donner l'acide correspondant de formule Ie₂ : 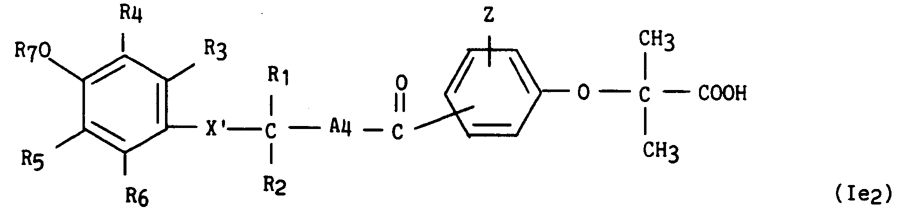 dans laquelle R₁, R₂, R₃, R₄, R₅, R₆, R₇, X', A₄ et Z ont les singifications précédemment définies ;
f) soit le composé de formule Ie₁ précédemment défini avec un réducteur chimique tel que par exemple le borohydrure de sodium ou avec de l'hydrogène en présence d'un catalyseur d'hydrogénation pour obtenir le composé de formule If₁ : 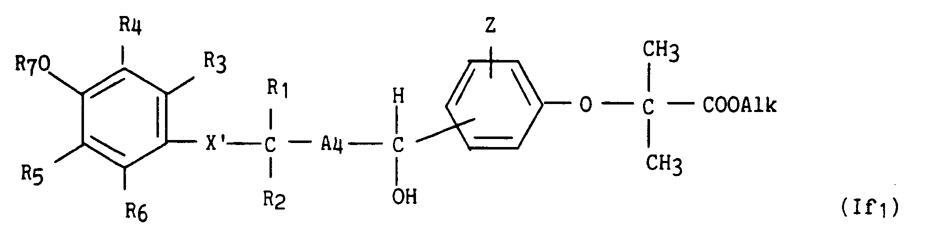 dans laquelle :
- R₁, R₂, R₃, R₄, R₅, R₆, R₇, X', A₄, Z et Alk ont les significations précédemment définies,
lequel composé de formule If₁ ci-dessus défini peut être saponifié pour donner l'acide correspondant de formule If₂ : 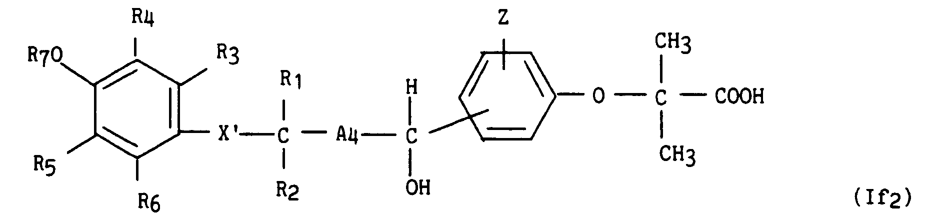 dans laquelle R₁, R₂, R₃, R₄, R₅, R₆, R₇, X', A₄ et Z ont les significations précédemment définies ;
g) soit un composé de formule If₁ ci-dessus défini,
avec un hydracide de formule IV :
H-Y (IV)
dans laquelle Y a la signification précédemment définie,
pour obtenir un composé de formule Ig₁ : 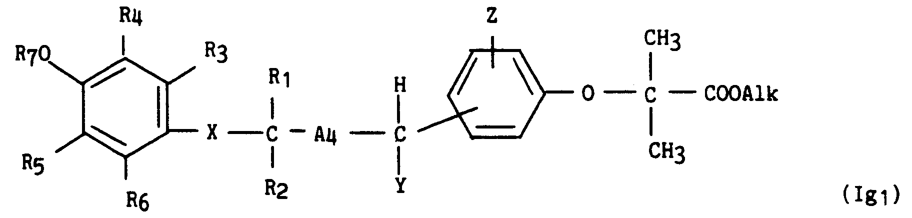 dans laquelle R₁, R₂, R₃, R₄, R₅, R₆, R₇, A₄, Y, Z et Alk ont les significations précédemment définies,
lequel composé Ig₁ ci-dessus défini est hydrolysé pour donner l'acide correspondant de formule Ig₂ : 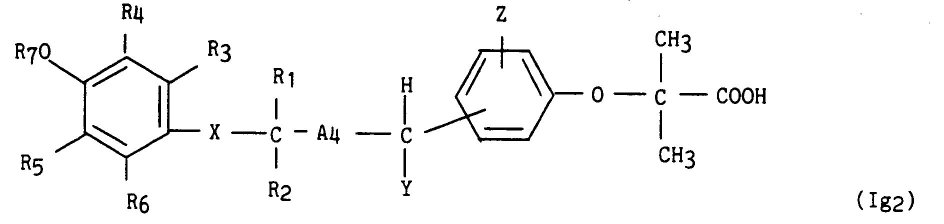 dans laquelle R₁, R₂, R₃, R₄, R₅, R₆, R₇, A₄, Y et Z ont les significations précédemment définies.
L'ensemble des composés de formule Ia₁, Ia₂, Ib₁, Ib₂, Ic₁, Ic₂, Ic₃, Id₁, Id₂, Ie₁, Ie₂, If₁, If₂, Ig₁, Ig₂ forme l'ensemble des composés de formule I.

11. Les compositions pharmaceutiques contenant comme principe actif un dérivé selon l'une quelconque des revendications 1 à 9 avec des excipients pharmaceutiques appropriés.

12. Les compositions pharmaceutiques selon la revendication 11 présentées sous une forme convenant pour le traitement des hypercholestéromémies, des hypertriglycéridémies, des dyslipémies et du diabète, de l'athérosclérose et des pathologies dans lesquelles une peroxydation lipidique membranaire joue un rôle initiateur et/ou aggravant.

## Claims

1. Substituted phenoxyisobutyric acids and esters of formula I: 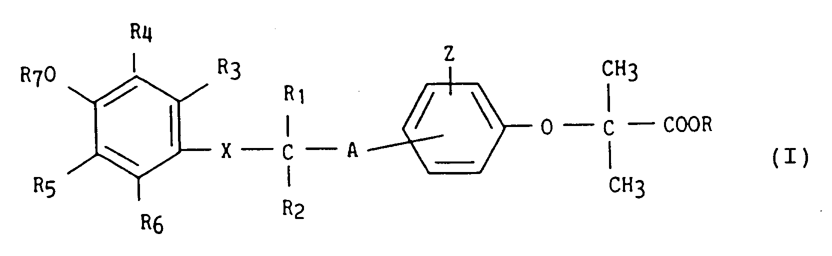 wherein:
- X represents an oxygen atom, a sulphur atom or a single bond;
- A represents a single bond or a hydrocarbon radical containing from 1 to 9 carbon atoms in a straight or branched chain optionally including a double bond, a cyclopropyl radical, an oxygen atom or a carbonyl radical, or optionally substituted by a halogen atom or a hydroxy radical;
- R represents a hydrogen atom or an alkyl radical that has from 1 to 6 carbon atoms in a straight or branched chain and is optionally substituted by one or two hydroxy radicals;
- R₁ and R₃:
. each simultaneously represents a hydrogen atom, or
. together form a (CH₂)ₙ bridge wherein n is 1 or 2, or
. R₁ represents:
. a methyl radical, or
. a single bond forming a double bond with the group A when that group is a hydrocarbon radical, and
. in each of those cases, R₃ simultaneously represents a hydrogen atom;
- each of R₂ and R₆, which may be identical or different, represents a hydrogen atom or a methyl radical;
- each of R₄ and R₅, which may be identical or different, represents an alkyl radical having from 1 to 6 carbon atoms in a straight or branched chain;
- R₇ represents a hydrogen atom or a labile protecting grouping, such as, for example, a CH₃CO-, C₂H₅O-CH₂- or benzyl radical; and
- Z represents a hydrogen or halogen atom or an alkyl or alkoxy radical each containing from 1 to 5 carbon atoms in a straight or branched chain;
and, when they exist, the corresponding enantiomers and diastereoisomers,
and also the physiologically tolerable salts of the compounds I with suitable bases.

2. 2-{4-[2-(3,5-di-tert-butyl-4-hydroxyphenylthio)ethyl]phenoxy}isobutyric acid.

3. 2-{4-[2-(4-hydroxy-2,3,5-trimethylphenylthio)ethyl]phenoxy}isobutyric acid.

4. 2-[4-(3,5-di-tert-butyl-4-hydroxyphenylthiomethyl)phenoxy]isobutyric acid.

5. The tert-butylamine salt of 2-{4-[3-(3,5-di-tert-butyl-4-hydroxyphenylthio)propyl]phenoxy}isobutyric acid.

6. 2-{4-[2-(4-hydroxy-2,3,5-trimethylphenoxy)ethyl]phenoxy}isobutyric acid.

7. 2-{4-[2-(3,5-di-tert-butyl-4-hydroxyphenoxy)ethyl]phenoxy}isobutyric acid.

8. Tert-butylamine (Z)-(R,S)-2-{4-[3-(6-hydroxy-2,5,7,8-tetramethylchroman-2-yl)prop-2-en-1-yl]phenoxy}isobutyrate.

9. Tert-butylamine (R,S)-2-{4-[3-(6-hydroxy-2,5,7,8-tetramethylchroman-2-yl)propyl]phenoxy}isobutyrate.

10. Process for the preparation of the compounds of claim 1, characterised in that:
a)
. a compound of formula IIa: 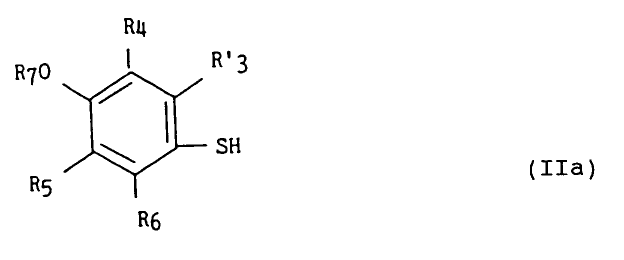 wherein:
- R₄, R₅, R₆ and R₇ are as defined in claim 1, and
- R'₃ represents a hydrogen atom,
is reacted
. with a compound of formula IIIa: 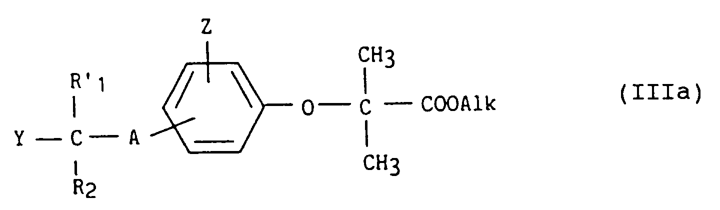 wherein:
- R₂, A and Z are as defined in claim 1;
- R'₁ represents a hydrogen atom or a methyl radical;
- Alk represents an alkyl radical having from 1 to 6 carbon atoms in a straight or branched chain, and
- Y represents a chlorine or bromine atom;
. to obtain a compound of formula Ia₁: 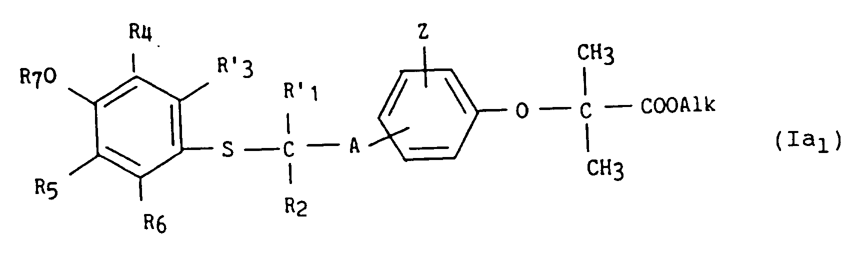 wherein R'₁, R₂, R'₃, R₄, R₅, R₆, R₇, A, Z and Alk are as defined above,
which compound Ia₁ is, depending on the nature of R₇, converted by sequential methods of saponification, hydrolysis or hydrogenolysis into a compound of formula Ia₂: 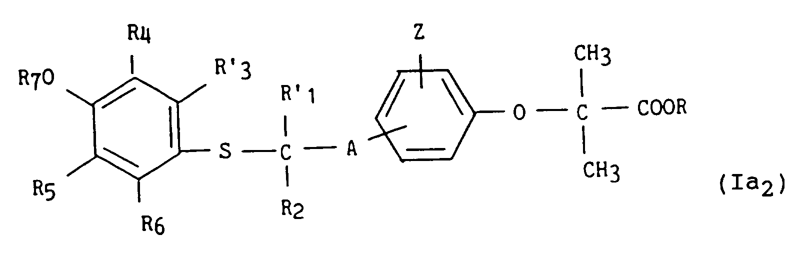 wherein R'₁, R₂, R'₃, R₄, R₅, R₆, R₇, A, Z and R are as defined in claim 1, or
b)
. a compound of formula IIb: 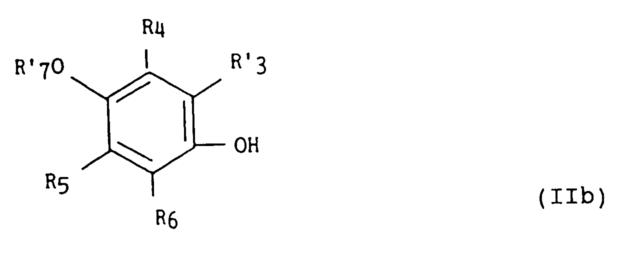 wherein:
- R'₃, R₄, R₅ and R₆ are as defined above and
- R'₇ represents a labile protecting grouping, such as: CH₃CO-, C₂H₅-O-CH₂- or benzyl;
is reacted
. with a compound of formula IIIb: 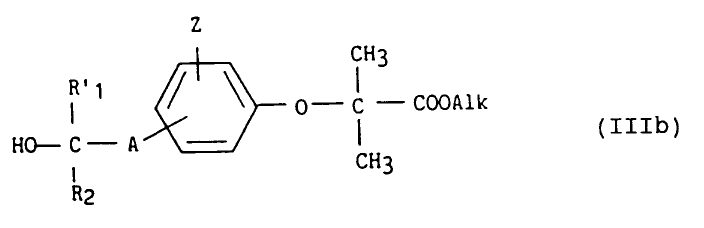 wherein R'₁, R₂, A, Z and Alk are as defined above,
. to obtain a compound of formula Ib₁: 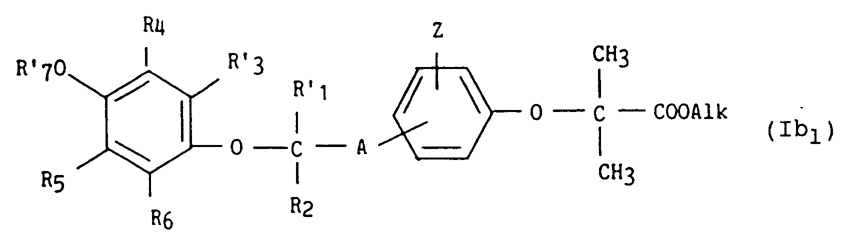 wherein R'₁, R₂, R'₃, R₄, R₅, R₆, R'₇, A, Z and Alk are as defined above,
which compound of formula Ib₁ is, depending on the nature of R'₇, converted by sequential methods of saponification, hydrolysis or hydrogenolysis into a compound of formula Ib₂: 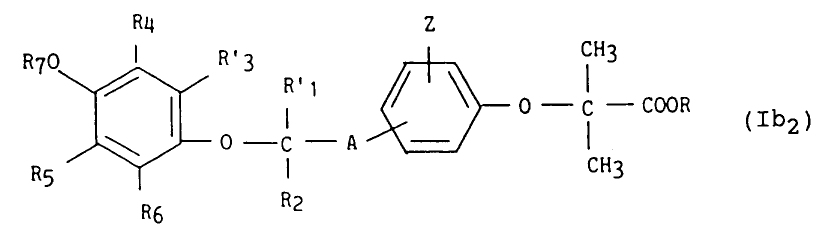 wherein R'₁, R₂, R'₃, R₄, R₅, R₆, R₇, A, Z and R are as defined above; or
c)
. a compound of formula IIc: 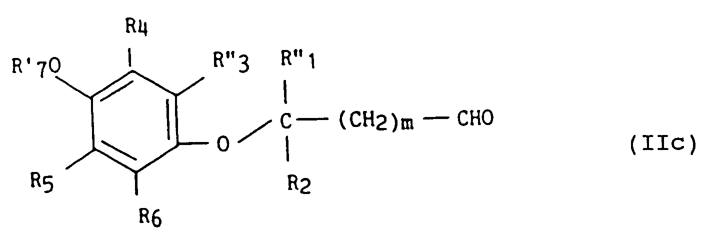 wherein:
- R₂, R₄, R₅, R₆ and R'₇ are as defined above,
- R''₁ and R''₃ together represent a (CH₂)ₙ bridge wherein n is as defined in claim 1, and
- m represents zero or an integer from 1 to 5;
is reacted
. with a compound of formula IIIc: 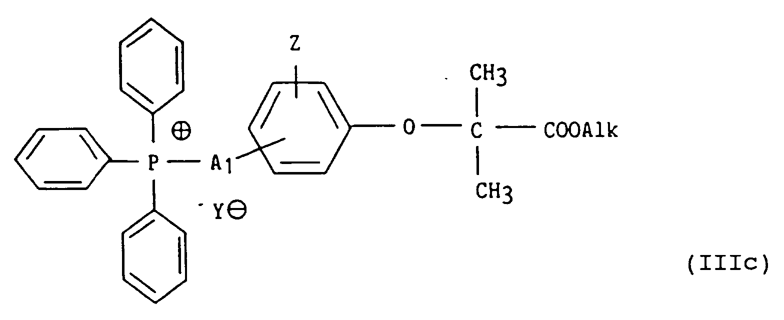 wherein:
- Alk and Z and Y are as defined above,
- A₁ represents a single bond or a hydrocarbon radical containing from 1 to 3 carbon atoms in a straight or branched chain;
. to obtain a compound of formula Ic₁: 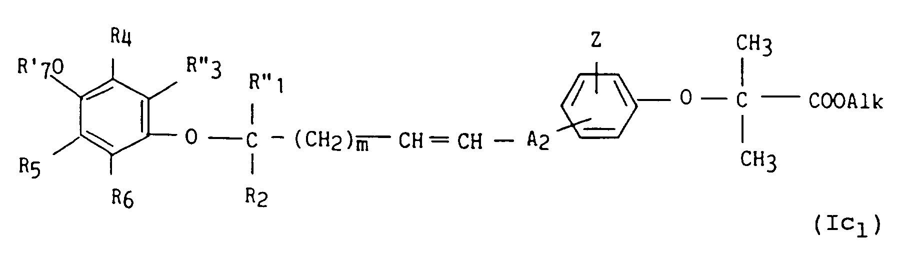 wherein:
- R''₁, R₂, R''₃, R₄, R₅, R₆, R'₇, m, Z and Alk are as defined above, and
- A₂ represents a single bond or a hydrocarbon radical containing 1 or 2 carbon atoms in a straight or branched chain;
which compound of formula Ic₁ is reduced to obtain the compound of formula Ic₂: 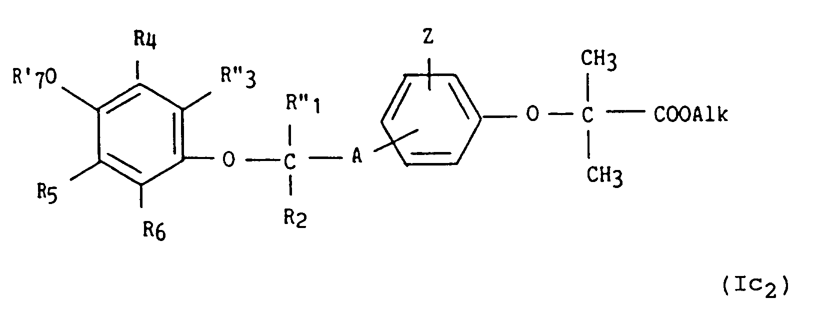 wherein R''₁, R₂, R''₃, R₄, R₅, R₆, R'₇, A, Z and Alk are as defined above;
which compound of formula Ic₂ is, depending on the nature of R'₇, converted by sequential methods of saponification, hydrolysis or hydrogenolysis into a compound of formula Ic₃: 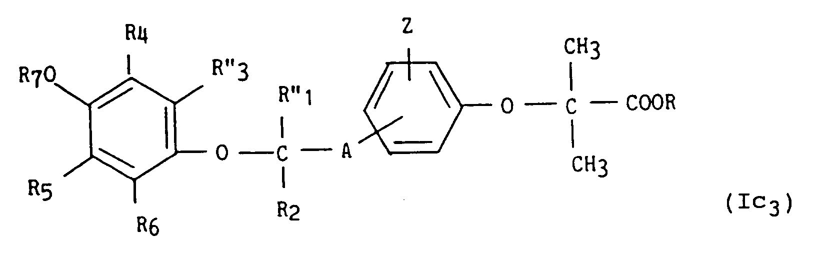 wherein R''₁, R₂, R''₃, R₄, R₅, R₆, R₇, A, Z and R are as defined above; or
d)
. a compound of formula IId): 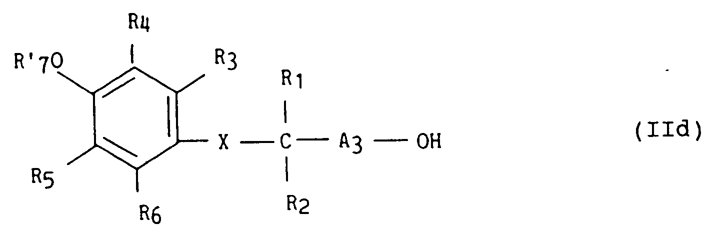 wherein:
- R₁, R₂, R₃, R₄, R₅, R₆, R'₇ and X are as defined above, and
- A₃ represents a single bond or a hydrocarbon radical containing from 1 to 9 carbon atoms in a straight or branched chain, is reacted
. with a compound of formula IIId: 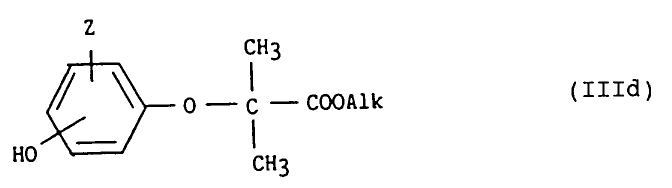 wherein Z and Alk are as defined above,
. to obtain a compound of formula Id₁: 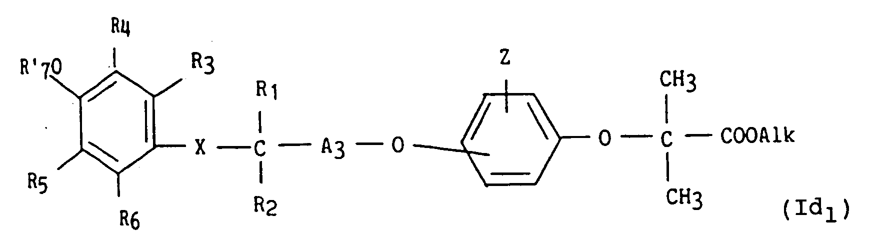 wherein R₁, R₂, R₃, R₄, R₅, R₆, R'₇, X, A₃ and Alk are as defined above;
which compound of formula Id₁ defined above may be saponified, hydrogenolysed or hydrolysed to give the corresponding acid of formula Id₂: 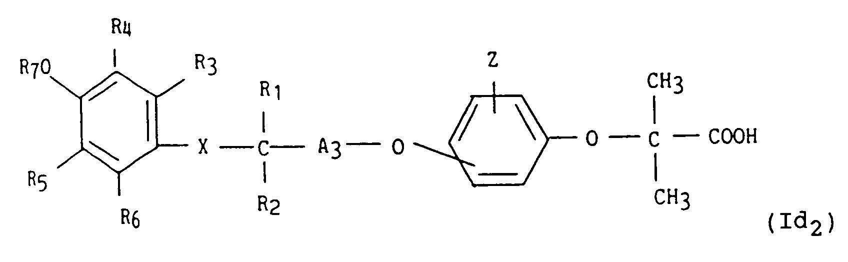 wherein R₁, R₂, R₃, R₄, R₅, R₆, R₇, X, A₃ and Z are as defined above; or
e)
. a compound of formula IIe: 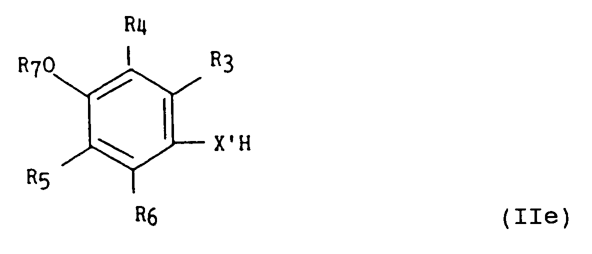 wherein:
- R₃, R₄, R₅, R₆ and R₇ are as defined above, and
- X' represents an oxygen or sulphur atom, is reacted
. with a compound of formula IIIe: 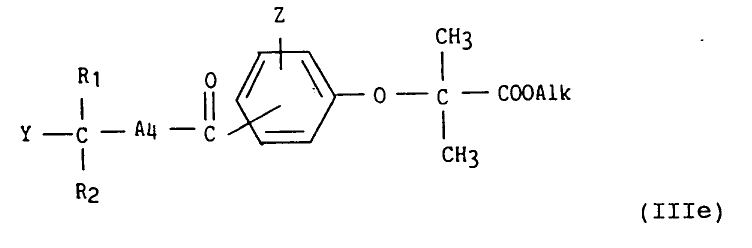 wherein:
- Y, R₁, R₂, Z and Alk are as defined above, and
- A₄ represents a single bond or a hydrocarbon chain having from 1 to 8 carbon atoms;
. to obtain a compound of formula Ie₁: 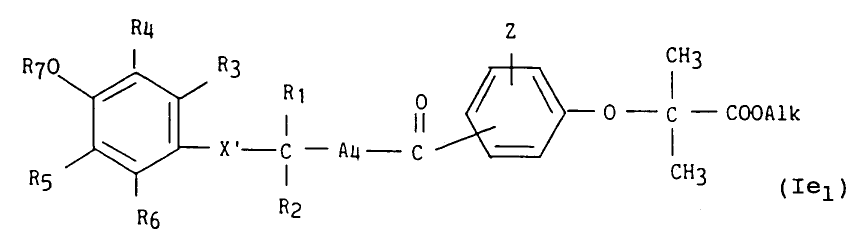 wherein R₁, R₂, R₃, R₄, R₅, R₆, R₇, X', A₄, Z and Alk are as defined above;
which compound of formula Ie₁ defined above may be saponified to give the corresponding acid of formula Ie₂: 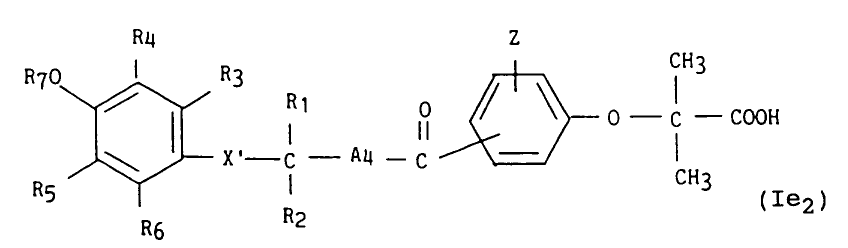 wherein R₁, R₂, R₃, R₄, R₅, R₆, R₇, X', A₄ and Z are as defined above; or
f)
. the compound of formula Ie₁ defined above is reacted with a chemical reducing agent, such as, for example, sodium borohydride, or with hydrogen in the presence of a hydrogenation catalyst to obtain the compound of formula If₁: 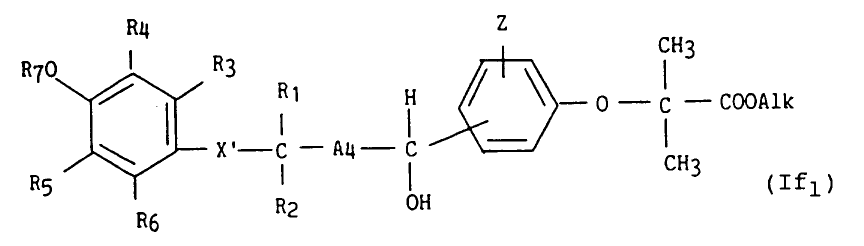 wherein:
- R₁, R₂, R₃, R₄, R₅, R₆, R₇, X', A₄, Z and Alk are as defined above;
which compound of formula If₁ defined above may be saponified to give the corresponding acid of formula If₂: 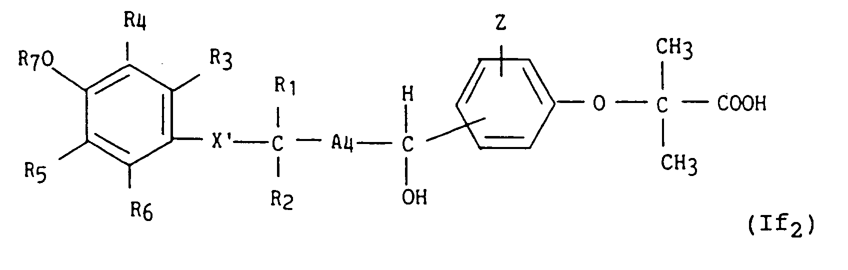 wherein R₁, R₂, R₃, R₄, R₅, R₆, R₇, X', A₄ and Z are as defined above; or
g)
. a compound of formula If₁ defined above is reacted
. with a hydracid of formula IV:
H-Y (IV)
wherein Y is as defined above,
. to obtain a compound of formula Ig₁: 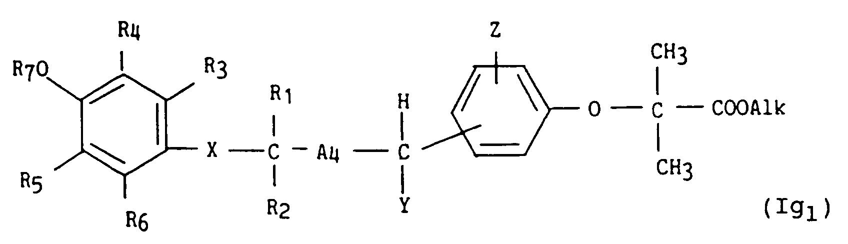 wherein R₁, R₂, R₃, R₄, R₅, R₆, R₇, A₄, Y, Z and Alk are as defined above,
which compound Ig₁ defined above is hydrolysed to give the corresponding acid of formula Ig₂: 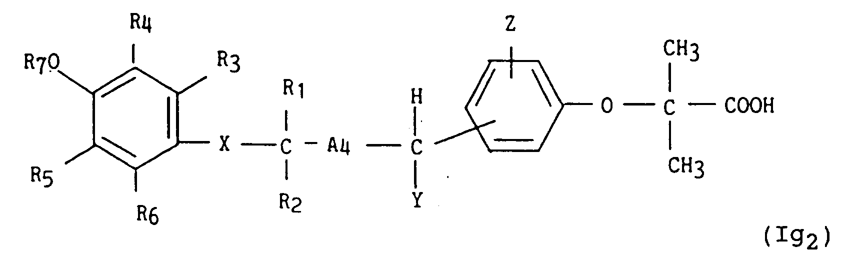 wherein R₁, R₂, R₃, R₄, R₅, R₆, R₇, A₄, Y and Z are as defined above.
The totality of the compounds of formulae Ia₁, Ia₂, Ib₁, Ib₂, Ic₁, Ic₂, Ic₃, Id₁, Id₂, Ie₁, Ie₂, If₁, If₂, Ig₁ and Ig₂ form the totality of the compounds of formula I.

11. Pharmaceutical compositions comprising as active ingredient a compound according to any one of claims 1 to 9 with suitable pharmaceutical excipients.

12. Pharmaceutical compositions according to claim 11 presented in a form suitable for the treatment of hypercholesterolaemia, hypertriglyceridaemia, dyslipaemia and diabetes, atherosclerosis and pathologies in which membrane lipid peroxidation plays an initiating and/or aggravating role.

## Patentansprüche

1. Phenoxy-isobuttersäuren und -ester der Formel I: 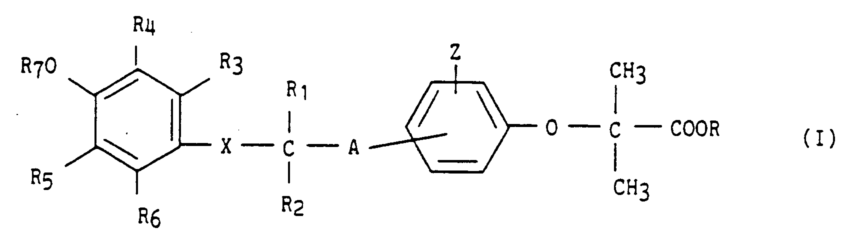 in der:
- X ein Sauerstoffatom, ein Schwefelatom oder eine Einfachbindung;
- A eine Einfachbindung oder eine Kohlenwasserstoffgruppe mit 1 bis 9 Kohlenstoffatomen in gerader oder verzweigter Kette, welche gegebenenfalls eine Doppelbindung, eine Cyclopropylgruppe, ein Sauerstoffatom oder eine Carbonylgruppe aufweist oder gegebenenfalls durch ein Halogenatom oder eine Hydroxylgruppe substituiert ist;
- R ein Wasserstoffatom oder eine Alkylgruppe mit 1 bis 6 Kohlenstoffatomen in gerader oder verzweigter Kette, die gegebenenfalls durch eine oder zwei Hydroxylgruppen substituiert ist;
- R₁ und R₃:
. jeweils gleichzeitig ein Wasserstoffatom darstellen oder
. gemeinsam eine Brücke (CH₂)ₙ, worin n die Werte 1 oder 2 besitzt, bilden, oder
. R₁:
. eine Methylgruppe oder
. eine Einfachbindung, die mit der Gruppe A, wenn diese eine Kohlenwasserstoffgruppe darstellt, eine Doppelbindung bildet und
. in jedem dieser Fälle R₃ gleichzeitig ein Wasserstoffatom darstellt;
- R₂ und R₆, die gleichartig oder verschieden sein können, jeweils ein Wasserstoffatom oder eine Methylgruppe;
- R₄ und R₅, die gleichartig oder verschieden sein können, jeweils eine Alkylgruppe mit 1 bis 6 Kohlenstoffatomen in gerader oder verzweigter Kette;
- R₇ ein Wasserstoffatom oder eine labile Schutzgruppe, wie beispielsweise eine CH₃CO-, C₂H₅O-CH₂- oder Benzylgruppe; und
- Z ein Wasserstoffatom, ein Halogenatom oder eine Alkyl- oder Alkoxygruppe mit jeweils 1 bis 5 Kohlenstoffatomen in gerader oder verzweigter Kette bedeuten,
und, falls sie existieren, deren entsprechende Enantiomere und Diastereoisomere,
sowie die physiologisch verträglichen Salze der Verbindungen I mit geeigneten Basen.

2. 2-{4-[2-(3,5-Di-tert.-butyl-4-hydroxyphenylthio)-ethyl]-phenoxy}-isobuttersäure.

3. 2-{4-[2-(4-Hydroxy-2,3,5-trimethylphenylthio)-ethyl]-phenoxy}-isobuttersäure.

4. 2-[4-(3,5-Di-tert.-butyl-4-hydroxyphenylthiomethyl)-phenoxy]-isobuttersäure.

5. 2-{4-[3-(3,5-Di-tert.-butyl-4-hydroxyphenylthio)-propyl]-phenoxy}-isobuttersäure-tert.-butylaminsalz.

6. 2-{4-[2-(4-Hydroxy-2,3,5-trimethylphenoxy)-ethyl]-phenoxy}-isobuttersäure.

7. 2-{4-[2-(3,5-Di-tert.-butyl-4-hydroxyphenoxy)-ethyl]-phenoxy}-isobuttersäure.

8. (Z),(R,S)-2-{4-[3-(6-Hydroxy-2,5,7,8-tetramethylchroman-2-yl)-prop-2-en-1-yl]-phenoxy}-isobuttersäure-tert.-butylaminsalz.

9. (R,S)-2-{4-[3-(6-Hydroxy-2,5,7,8-tetramethylchroman-2-yl)-propyl]-phenoxy}-isobuttersäure-tert.-butylaminsalz.

10. Verfahren zur Herstellung der Verbindungen nach Anspruch 1, dadurch gekennzeichnet, daß man:
a)
. entweder eine Verbindung der Formel IIa: 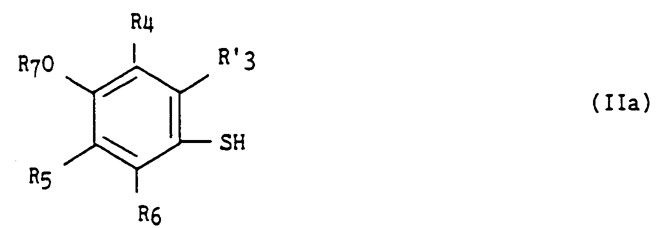 in der:
- R₄, R₅, R₆ und R₇ die in Anspruch 1 angegebenen Bedeutungen besitzen und
- R'₃ ein Wasserstoffatom bedeutet,
. mit einer Verbindung der Formel IIIa: 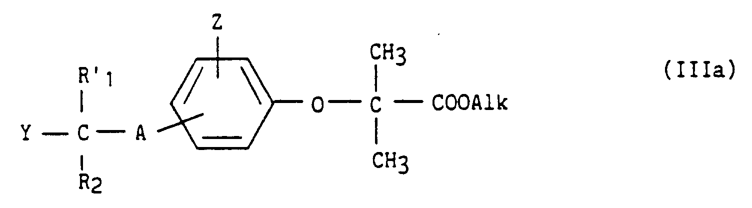 in der:
- R₂, A und Z die in Anspruch 1 angegebenen Bedeutungen besitzen;
- R'₁ ein Wasserstoffatom oder eine Methylgruppe darstellt;
- Alk eine Alkylgruppe mit 1 bis 6 Kohlenstoffatomen in gerader oder verzweigter Kette bedeutet
und
- Y ein Chloratom oder ein Bromatom bedeutet; umsetzt
. zur Bildung einer Verbindung der Formel Ia₁: 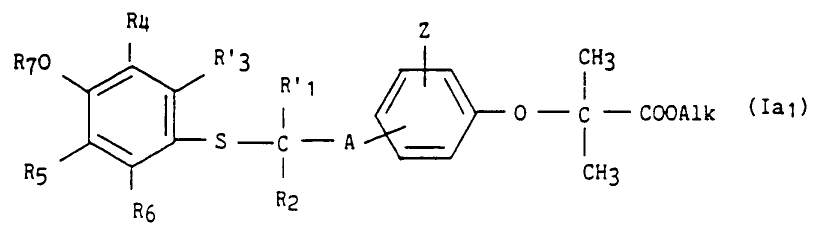 in der R'₁, R₂, R'₃, R₄, R₅, R₆, R₇, A, Z und Alk die oben angegebenen Bedeutungen besitzen,
welche Verbindung Ia₁ in Abhängigkeit von der Art der Gruppe R₇ durch aufeinanderfolgende Methoden der Verselfung, Hydrolyse oder Hydrogenolyse in eine Verbindung der Formel Ia₂ umgewandelt wird: 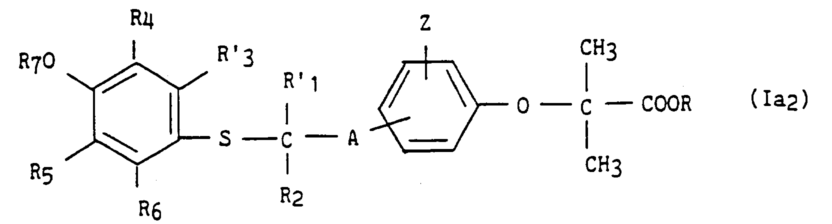 in der R'₁, R₂, R'₃, R₄, R₅, R₆, R₇, A, Z und R die in Anspruch 1 angegebenen Bedeutungen besitzen,
b)
. oder eine Verbindung der Formel IIb: 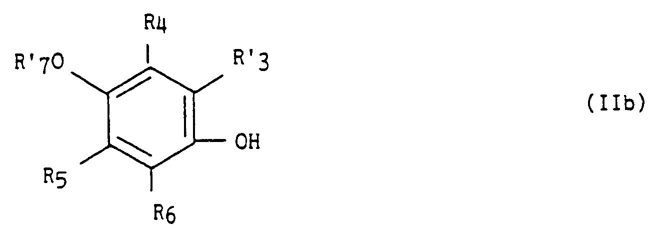 in der:
- R'₃, R₄, R₅ und R₆ die oben angegebenen Bedeutungen besitzen und
- R'₇ eine labile Schutzgruppe darstellt, wie eine CH₃CO-, C₂H₅-O-CH₂- oder Benzylgruppe bedeutet;
. mit einer Verbindung der Formel IIIb: 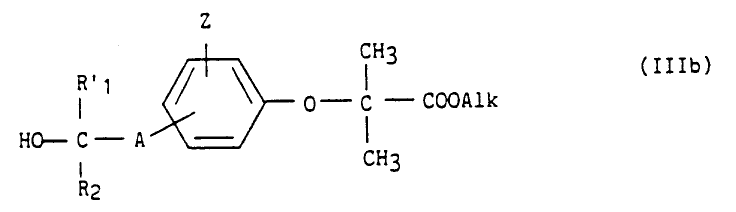 in der R'₁, R₂, A, Z und Alk die oben angegebenen Bedeutungen besitzen, umsetzt
. zur Bildung einer Verbindung der Formel Ib₁: 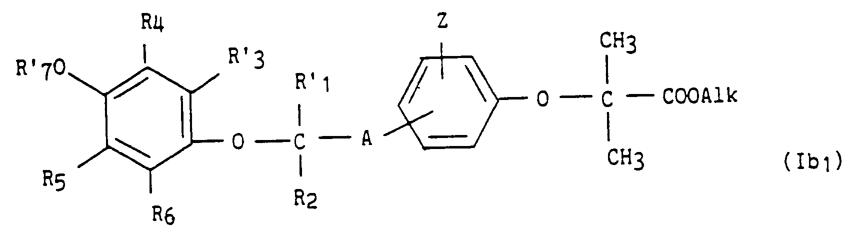 in der R'₁, R₂, R'₃, R₄, R₅, R₆, R'₇, A, Z und Alk die oben angegebenen Bedeutungen besitzen,
. welche Verbindung der Formel Ib₁ in Abhängigkeit von der Art der Gruppe R'₇ mit Hilfe aufeinanderfolgender Methoden der Verseifung, Hydrolyse oder Hydrogenolyse in eine Verbindung der Formel Ib₂ umgewandelt wird: 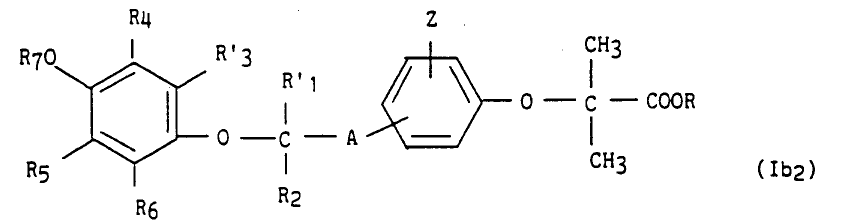 in der R'₁, R₂, R'₃, R₄, R₅, R₆, R₇, A, Z und R die oben angegebenen Bedeutungen besitzen;
c)
. oder eine Verbindung der Formel IIc: 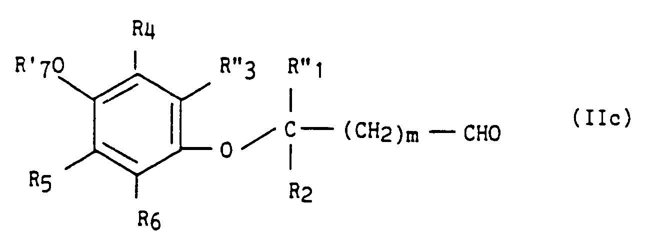 in der:
- R₂, R₄, R₅, R₆ und R'₇ die oben angegebenen Bedeutungen besitzen,
- R''₁ und R''₃ gemeinsam eine Brücke (CH₂)ₙ, worin n die in Anspruch 1 angegebenen Bedeutungen besitzt, bilden, und
- m 0 oder eine ganze Zahl mit einem Wert von 1 bis 5 darstellt;
. mit einer Verbindung der Formel IIIc; 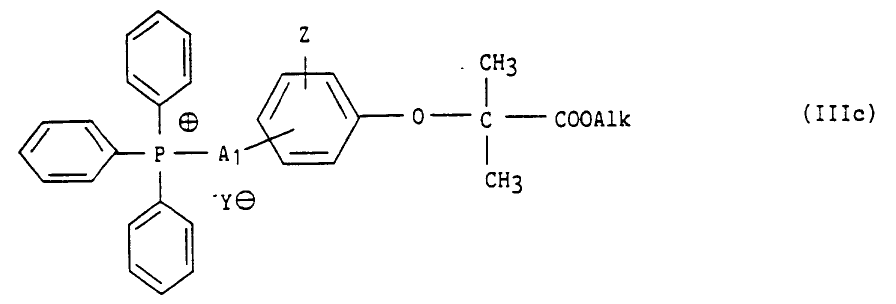 in der:
- Alk, Z und Y die oben angegebenen Bedeutungen besitzen und
- A₁ eine Einfachbindung oder eine Kohlenwasserstoffgruppe mit 1 bis 3 Kohlenstoffatomen in gerader oder verzweigter Kette bedeutet; umsetzt
. zur Bildung einer Verbindung der Formel Ic₁: 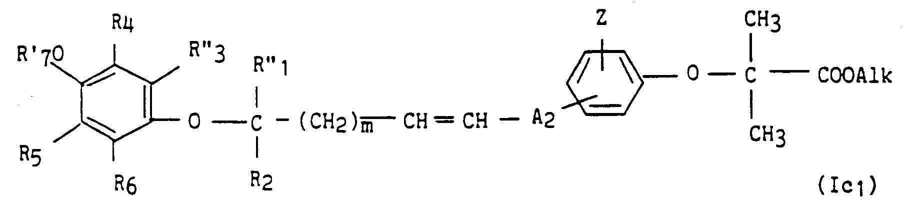 in der:
- R''₁, R₂, R''₃, R₄, R₅, R₆, R₇, m, Z und Alk die oben angegebenen Bedeutungen besitzen und
- A₂ eine Einfachbindung oder eine Kohlenwasserstoffgruppe mit 1 oder 2 Kohlenstoffatomen in gerader oder verzweigter Kette bedeutet;
welche Verbindung der Formel Ic₁ reduziert wird zur Bildung der Verbindung der Formel Ic₂: 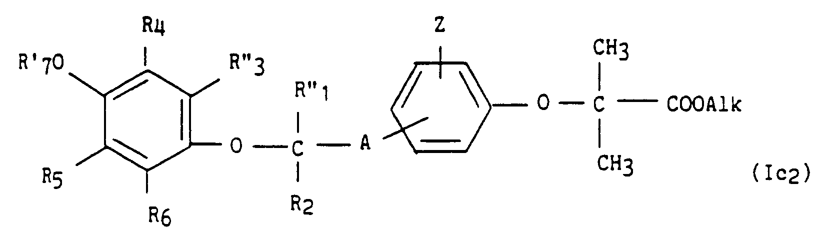 in der R''₁, R₂, R''₃, R₄, R₅, R₆, R'₇, A, Z und Alk die oben angegebenen Bedeutungen besitzen;
welche Verbindung der Formel Ic₂ in Abhängigkeit von der Art der Gruppe R'₇ mit Hilfe aufeinanderfolgender Methoden der Verseifung. Hydrolyse oder Hydrogenolyse in eine Verbindung der Formel Ic₃ umgewandelt wird: 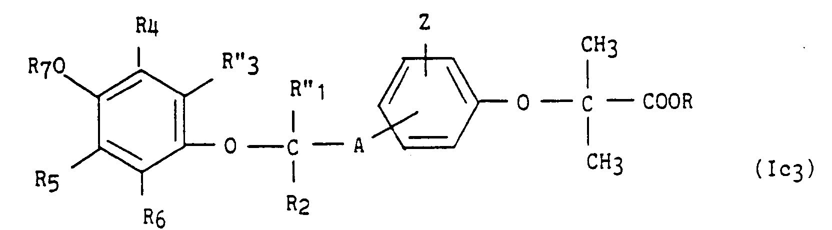 in der R''₁, R₂, R''₃, R₄, R₅, R₆, R₇, A, Z und R die oben angegebenen Bedeutungen besitzen;
d) oder eine Verbindung der Formel IId): 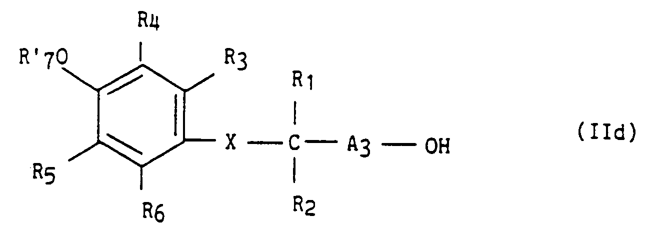 in der:
- R₁, R₂, R₃, R₄, R₅, R₆, R'₇ und X die oben angegebenen Bedeutungen besitzen und
- A₃ eine Einfachbindung oder eine Kohlenwasserstoffgruppe mit 1 bis 9 Kohlenstoffatomen in gerader oder verzweigter Kette darstellt,
mit einer Verbindung der Formel IIId: 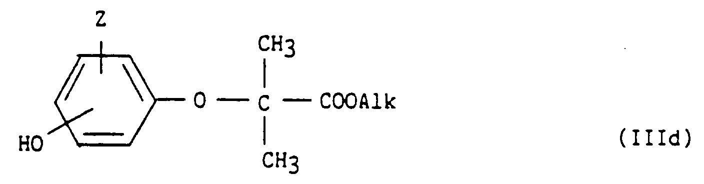 in der Z und Alk die oben angegebenen Bedeutungen besitzen, umsetzt,
zur Bildung einer Verbindung der Formel Id₁: 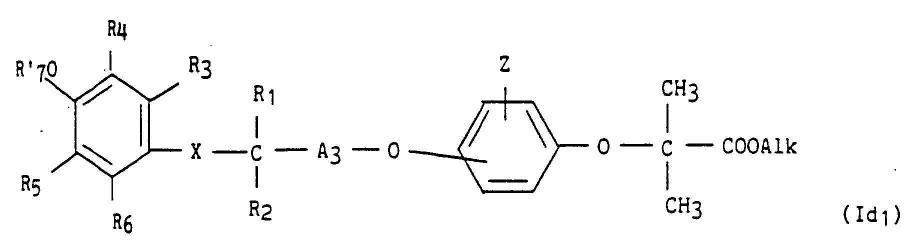 in der R₁, R₂, R₃, R₄, R₅, R₆, R'₇, X, A₃ und Alk die oben angegebenen Bedeutungen besitzen;
welche Verbindung der oben definierten Formel Id₁ verseift, hydrogenolysiert oder hydrolyslert werden kann zur Bildung der entsprechenden Säure der Formel Id₂: 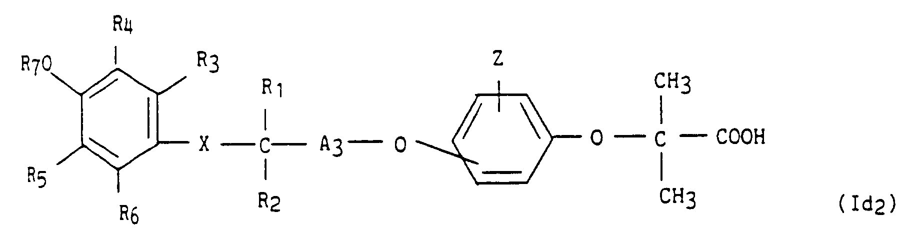 in der R₁, R₂, R₃, R₄, R₅, R₆, R₇, X, A₃ und Z die oben angegebenen Bedeutungen besitzen;
e) oder eine Verbindung der Formel IIe: 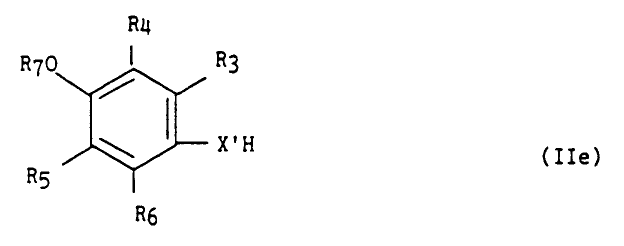 in der:
- R₃, R₄, R₅, R₆ und R₇ die oben angegebenen Bedeutungen besitzen und
- X' ein Sauerstoffatom oder ein Schwefelatom darstellt,
mit einer Verbindung der Formel IIIe: 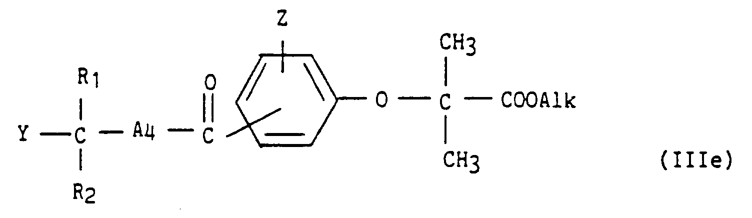 in der:
- Y, R₁, R₂, Z und Alk die oben angegebenen Bedeutungen besitzen und
- A₄ eine Einfachbindung oder eine Kohlenwasserstoffkette mit 1 bis 8 Kohlenstoffatomen darstellt; umsetzt
zur Bildung einer Verbindung der Formel Ie₁: 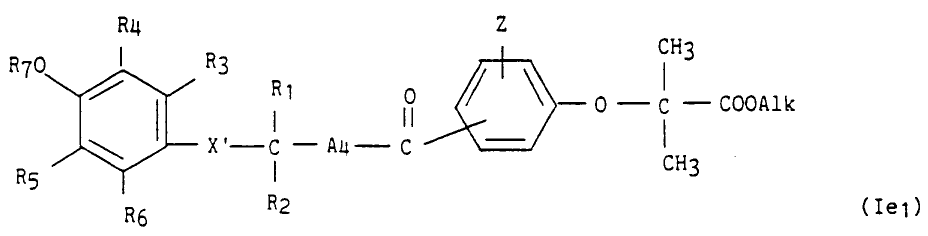 in der R₁, R₂, R₃, R₄, R₅, R₆, R₇, X', A₄, Z und Alk die oben angegebenen Bedeutungen besitzen;
welche Verbindung der oben definierten Formel Ie₁ verseift werden kann zur Bildung der entsprechenden Säure der Formel Ie₂: 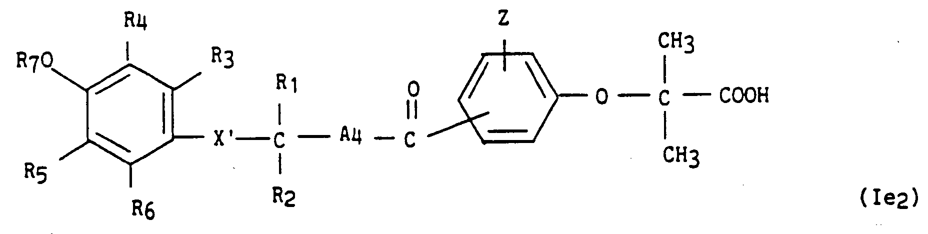 in der R₁, R₂, R₃, R₄, R₅, R₆, R₇, X', A₄ und Z die oben angegebenen Bedeutungen besitzen;
f) oder man setzt die Verbindung der oben definierten Formel Ie₁ mit einem chemischen Reduktionsmittel, wie beispielsweise Natriumborhydrid oder mit Wasserstoff in Gegenwart eines Hydrierkatalysators um zur Bildung der Verbindung der Formel If₁: 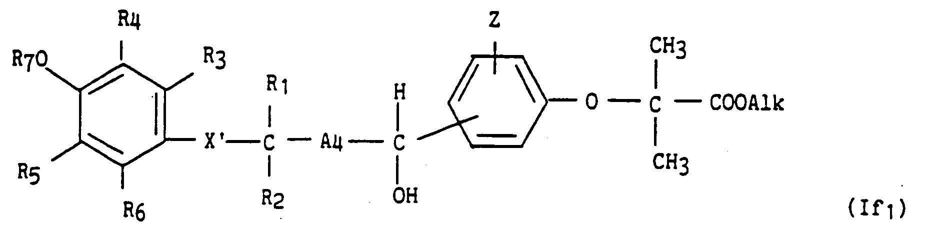 in der:
- R₁, R₂, R₃, R₄, R₅, R₆, R₇, X', A₄, Z und Alk die oben angegebenen Bedeutungen besitzen,
welche Verbindung der oben definierten Formel If₁ verseift werden kann zur Bildung der entsprechenden Säure der Formel If₂: 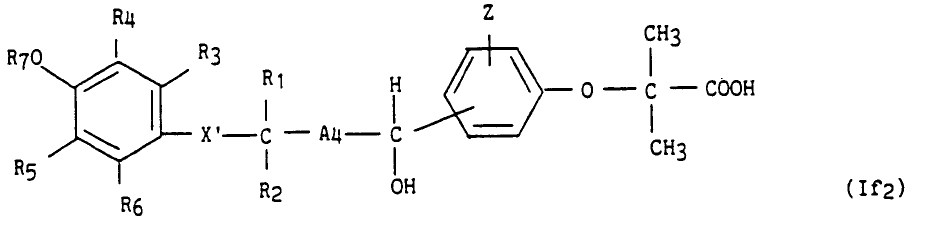 in der R₁, R₂, R₃, R₄, R₅, R₆, R₇, X', A₄ und Z die oben angegebenen Bedeutungen besitzen;
g) oder eine Verbindung der oben definierten Formel If₁
mit einer Wasserstoffsäure der Formel IV:
H - Y (IV)
in der Y die oben angegebenen Bedeutungen besitzt, umsetzt
zur Bildung einer Verbindung der Formel Ig₁: 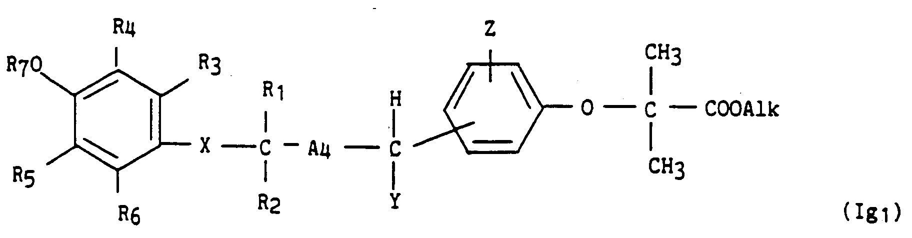 in der R₁, R₂, R₃, R₄, R₅, R₆, R₇, A₄, Y, Z und Alk die oben angegebenen Bedeutungen besitzen,
welche oben definierte Verbindung Ig₁ hydrolysiert wird zur Bildung der entsprechenden Säure der Formel Ig₂: 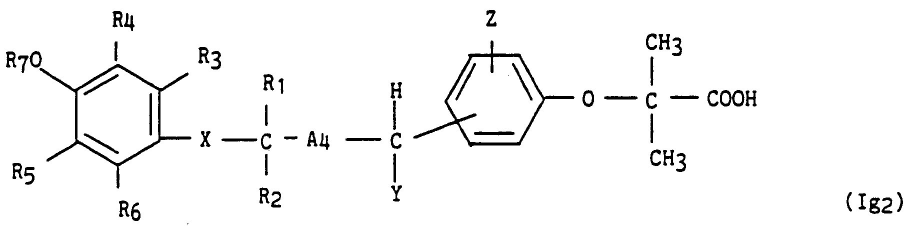 in der R₁, R₂, R₃, R₄, R₅, R₆, R₇, A₄, Y und Z die oben angegebenen Bedeutungen besitzen,
wobei die Gesamtheit der Verbindungen der Formeln Ia₁, Ia₂, Ib₁, Ib₂, Ic₁, Ic₂, Ic₃, Id₁, Id₂, Ie₁, Ie₂, If₁, If₂, Ig₁, Ig₂ die Gesamtheit der Verbindungen der Formel I bildet.

11. Pharmazeutische Zubereitung enthaltend als Wirkstoff ein Derivat nach irgendeinem der Ansprüche 1 bis 9 zusammen mit geeigneten pharmazeutischen Trägermaterialien.

12. Pharmazeutische Zubereitung nach Anspruch 11 in einer Form, welche für die Behandlung von Hypercholesterinämien. Hypertriglyceridämien, Dyslipidämien und des Diabetes, der Atherosklerose und von pathologischen Zuständen, bei denen eine Membranlipidperoxidation eine auslösende und/oder erschwerende Rolle spielt, geeignet ist.
